# FASCICULE DE BREVET EUROPEEN

(11) **EP 0 527 687 B1**
(45) Date de publication et mention de la délivrance du brevet: **22.11.1995**
(21) Numéro de dépôt: 92402279.1
(22) Date de dépôt: 13.08.1992
(51) Int. Cl.: C07D 209/16, C07D 235/08, C07D 231/56, C07D 261/20, C07D 307/81, C07D 333/58, A61K 31/34, A61K 31/38, A61K 31/405, A61K 31/415, A61K 31/42

(54) **Nouveaux dérivés d'aryléthylamines, leurs procédés de préparation et les compositions pharmaceutiques qui les contiennent**
Neue Arylethylaminderivate, Verfahren zu ihrer Herstellung und sie enthaltende pharmazeutische Zusammensetzungen
Novel arylethylamin derivatives, processes for their preparation and pharmaceutical compositions containing them

(30) Priorité: 13.08.1991 FR 9110261
(43) Date de publication de la demande: 17.02.1993
(73) Titulaire: ADIR ET COMPAGNIE, 92415 Courbevoie Cédex (FR)
(72) Inventeur: Lesieur, Daniel, F-59147 Gondecourt (FR); Yous, Said, Laboratoire de chimie Synthèse, F-59045 Lille Cedex (FR); Depreux, Patrick, F-59280 Armentières (FR); Andrieux, Jean, F-92160 Antony (FR); Adam, Gérard, F-78600 Le Mesnil Le Roi (FR); Caignard, Daniel Henri, F-75015 Paris (FR); Guardiola, Béatrice, F-92200 Neuilly Sur Seine (FR)

(56) Documents cités:
- WO-A-89/01472
- JOURNAL OF MEDICINAL CHEMISTRY vol. 22, no. 1, 1979, COLOMBUS, OHIO, US pages 63 - 69 M.E. FLAUGH ET AL. 'SYNTHESIS AND EVALUATION OF THE ANTIOVULATORY ACTIVITY OF A VARIETY OF MELATONIN ANALOGUES'
- JOURNAL OF HETEROCYCLIC CHEMISTRY vol. 20, 1983, TAMPA, FLORIDA, US pages 1697 - 1703 E. CAMPAIGNE ET AL. 'BENZO(B)THIOPHENE DERIVATIVES. XXVII. 5-METHOXY-6-HALO-3-BET A-ACETAMIDOETHYLBENZO(B)THIOPHENES, BLOCKED ANALOGS OF MELATONIN (1)

## Description

La présente invention concerne de nouveaux dérivés d'aryléthylamines, leurs procédés de préparation et les compositions pharmaceutiques qui les contiennent.

Un certain nombre de dérivés d'aryléthylamines possédant un noyau indolique sont décrits comme étant agonistes ou antagonistes de la mélatonine, que ce soit dans des brevets GB 219 2001, WO 89/01472, ou dans des publications J. Med. Chem (1979) 22 (1) p 63-69, et Chemical Abstract (1968) 70 (1) n° 3722 T.

Il en est de même pour quelques dérivés possédant un noyau benzo [b] thiophène : J. Med. Chem. (1970) 13 p 1205-1208, J. Heterocyclic Chem. (1978) 15 p 1351-1359, (1983) 20 p 1697-1703.

Des analogues benzo [b] furaniques de la mélatonine ont également été synthétisés : Annalen (1963) 662 pp 147-159, Brevet FR 1343073, mais aucune activité pharmacologique du type mélatoninomimétique ne semble avoir été recherchée.

Il en est de même en série benzimidazolique où des analogues déméthoxylés de la mélatonine ont été préparés sans qu'une telle activité semble avoir été recherchée : Khimiko Farmatsevticheskii Zhurnal (1968) 9 pp 21-23.

La demanderesse a présentement découvert de nouveaux dérivés possédant une affinité très largement supérieure à celle des produits décrits dans la littérature et de la mélatonine elle-même pour les récepteurs à la mélatonine.

Ces dérivés possèdent de nombreuses et intéressantes activités pharmacologiques de par leur caractère agoniste ou antagoniste de la mélatonine.

Outre leur action bénéfique sur les troubles du rythme circadien et du sommeil et les désordres saisonniers, ils possèdent d'intéressantes propriétés pharmacologiques sur le système nerveux central, notamment anxiolytiques, antipsychotiques, analgésiques, sur l'ovulation, la circulation cérébrale et l'immunomodulation.

Plus spécifiquement, la présente invention concerne les composés de formule générale **(I)** : 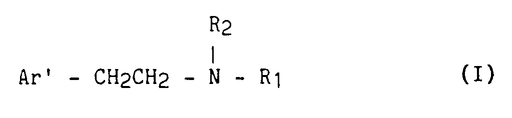
dans laquelle :
- Ar' représente :
   - un noyau indol-3-yl de formule **(II)** : 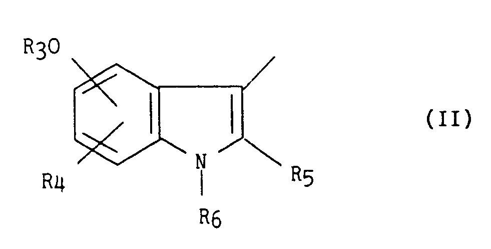
   - un noyau benzo [b] thiophèn-3-yl de formule **(III)** : 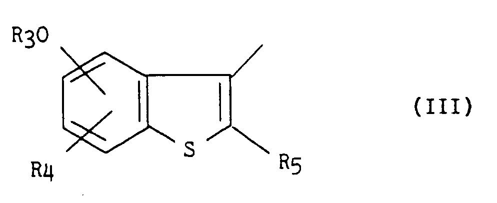
   - un noyau benzimidazol-1-yl de formule **(IV)** : 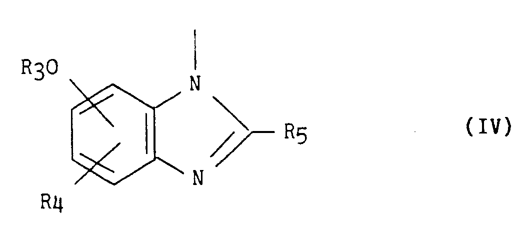
   - un noyau benzo [b] furan-3-yl de formule **(V)** : 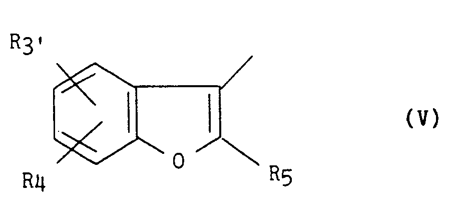
   - un noyau 1,2-benzisoxazol-3-yl de formule **(VI)** : 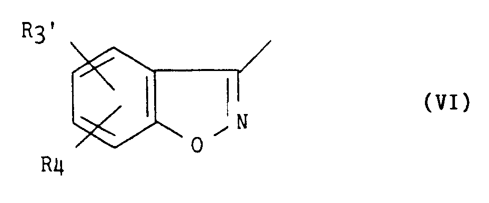
   - un noyau 1,2-benzisothiazol-3-yl de formule **(VII)** : 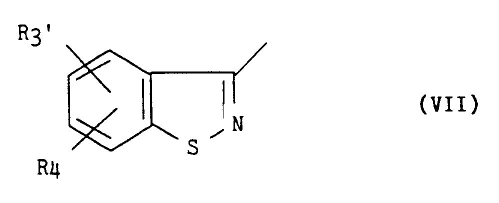
   - un noyau indazol-3-yl de formule **(VIII)** : 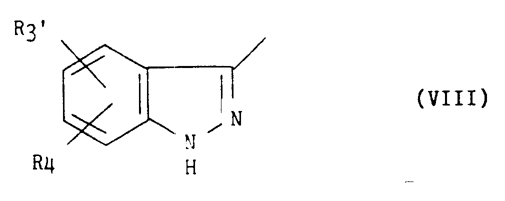
- R₁ représente :
   - un groupement 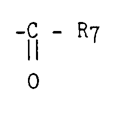 dans lequel R₇ représente un cycloalkyle éventuellement substitué, un cycloalkyl-(C₁-C₄)alkyle éventuellement substitué, ou un trifluorométhyle,
      étant entendu que lorsque Ar' représente un groupement choisi parmi ceux de formule (IV), (VI), (VII), et (VIII), alors R₇ peut également représenter un alkyle linéaire ou ramifié de 1 à 6 atomes de carbone non substitué ou substitué par 1 à 2 radicaux halogène,
   - un groupement 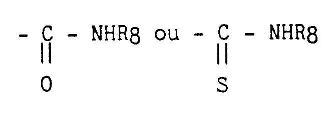 dans lesquels R₈ représente un alkyle inférieur de 1 à 6 atomes de carbone linéaire ou ramifié, un cycloalkyle éventuellement substitué, un cycloalkyl-(C₁-C₄)alkyle éventuellement substitué, un aryle éventuellement substitué, un arylalkyle éventuellement substitué dont la chaine alkyle comporte de 1 à 3 atomes de carbone,
   - un groupement 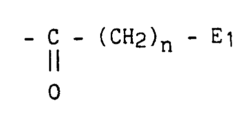 dans lequel
      n représente un nombre entier compris entre 1 et 3, et E₁ représente un radical choisi parmi :
   - morpholino,
   - pipérazine non substituée ou substituée par un radical -(CH₂)ₙ'-E₂ où n' représente un entier de 1 à 4 et E₂ représente un radical phényl ou naphtyl non substitué ou substitué par un à trois radicaux choisis parmi : halogène, (C₁-C₄) alkyl, et (C₁-C₄) alcoxy,
- R₂ représente un hydrogène ou un alkyle inférieur de 1 à 6 atomes de carbone linéaire ou ramifié,
- R₃ représente un hydrogène, un alkyle inférieur de 1 à 6 atomes de carbone linéaire ou ramifié, un aryle éventuellement substitué, un arylalkyle ou diarylalkyle éventuellement substitués dans lesquels la chaîne alkyle comporte de 1 à 3 atomes de carbone, ou un cycloalkyle ou un cycloalkylalkyl dans lequel la chaîne alkyle comporte de 1 à 3 atomes de carbone,
- R₃' représente un atome d'hydrogène ou un groupement -O-R₃ avec R₃ tel que défini précédemment,
- R₄ représente un hydrogène, un halogène, un hydroxy, un alcoxy de 1 à 6 atomes de carbone linéaire ou ramifié, un alkyle inférieur de 1 à 6 atomes de carbone linéaire ou ramifié,
- R₅ représente un hydrogène, un halogène, un alkyle inférieur de 1 à 6 atomes de carbone linéaire ou ramifié, un phényle éventuellement substitué, un phénylalkyle dont la chaîne alkyle comprend de 1 à 3 atomes de carbone, éventuellement substitué,
- R₆ représente un hydrogène, un alkyle inférieur de 1 à 6 atomes de carbone linéaire ou ramifié,
- leurs isomères, épimères, diastéréoisomères,
- et leurs sels d'addition à un acide ou à une base pharmaceutiquement acceptable,
- avec les réserves que :
   · Ar' ne peut pas représenter un groupement 7-méthoxy benzo [b] furan-3-yl lorsque R₁ représente un cyclopropylcarbonyle,
   · R₁ ne peut pas représenter un trifluoroacétyle, lorsque Ar' représente un indole avec R₂ = R₃ = R₄ = R₅ = R₆ = H,
   · R₁ ne peut pas représenter un radical anilinothiocarbonyl non substitué ou substitué en position 4 du phényl par un radical alcoxy lorsque Ar' représente un noyau indol-3-yl et R₃ représente un radical méthyle ou benzyle,
   · et R₁ ne peut pas représenter un groupement -CO-NH-R₈ lorsque Ar' représente un noyau de formule (II) et R₅ représente un phénylalkyle dont la chaîne alkyle comprend de 1 à 3 atomes de carbone, éventuellement substitué,
   étant entendu que, sauf précisions contraires :
- le terme "substitué" associé aux expressions "aryle", "arylalkyle", "diarylakyle", "phényle", et "phénylalkyle" signifie que le ou les noyaux aromatiques peuvent être substitués par un ou plusieurs radicaux choisis parmi : alkyle inférieur de 1 à 6 atomes de carbone linéaire ou ramifié, alcoxy inférieur de 1 à 6 atomes de carbone linéaire ou ramifié, hydroxy, halogène, nitro, et trifluorométhyle,
- le terme "substitué" associé aux expressions "cycloalkyle", et "cycloalkyl-(C₁-C₄)alkyle" signifie que le système cyclique peut être substitué par un ou plusieurs radicaux choisis parmi : halogène, alkyle inférieur de 1 à 6 atomes de carbone, linéaire ou ramifié et alcoxy inférieur de 1 à 6 atomes de carbone, linéaire ou ramifié,
- le terme "cycloalkyle" désigne un système cyclique, saturé ou insaturé, de 3 à 8 atomes de carbone,
- par groupement aryle on entend groupement pyridyle, phényle, naphtyle, thiényle, furyle, ou pyrimidyle.

La présente invention a également pour objet le procédé de préparation des composés de formule **(I)**, caractérisé en ce que l'on utilise comme matière première une amine de formule générale **(IX)** :

Ar' - CH₂CH₂ - NHR₂ **(IX)**

dans laquelle Ar' et R₂ ont la même signification que dans la formule **(I)**, que l'on traite :
- soit par un chlorure d'acide de formule **(X)** : 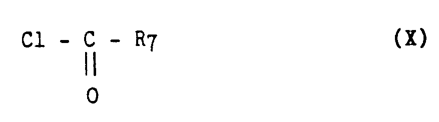 ou par l'anhydride d'acide correspondant de formule **(XI)** : 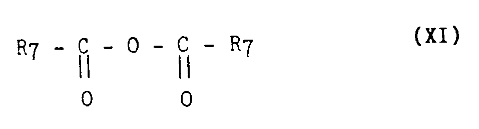 dans lesquelles R₇ a la même signification que dans la formule **(I)** de manière à obtenir les composés de formule **(Iα**) : 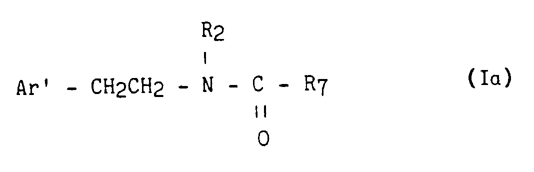 dans laquelle Ar', R₂ et R₇ ont la même signification que dans la formule **(I)**,
- soit par un isocyanate de formule **(XII)** :

   R₈ - N = C = O **(XII)**

   dans laquelle R₈ à la même signification que dans la formule **(I)** de manière à obtenir les composés de formule **(Iβ)**: 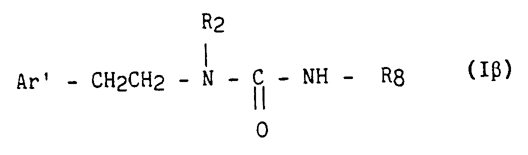 dans laquelle Ar', R₂ et R₈ ont la même signification que dans la formule **(I)**,
- soit par un isothiocyanate de formule **(XIII)** :

   R₈ - N = C = S **(XIII)**

   dans laquelle R₈ a la même signification que dans la formule **(I)** de manière à obtenir les composés de formule **(Iγ)** : 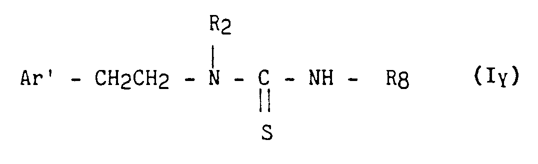 dans laquelle Ar', R₂ et R₈ ont la même signification que dans la formule **(I)** ,
   étant entendu que les composés de formules **(Iα)**, **(Iβ)** et **(Iγ)** font partie de l'invention et constituent les composés de formule **(I)**,
les composés de formule (I) pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

L'invention s'étend également au procédé d'obtention des composés de formule (Iε) : 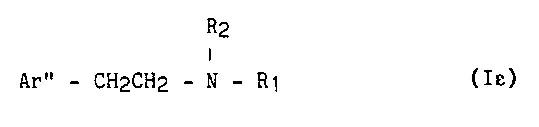
dans laquelle R₁ et R₂ sont tels que définis dans la formule (I) et Ar'' représente un groupement Ar' tel que défini dans la formule (I) substitué par un groupement -O-R₃'' avec R₃'' représentant un groupement choisi parmi aryle éventuellement substitué, arylalkyle ou diarylalkyle éventuellement substitués, ou cycloalkyle ou cycloalkylalkyle, (les termes : "aryle", "arylalkyle", "diarylalkyle", "cycloalkyle", "cycloalkylalkyle", et "substitué(s)" étant tels que définis dans la formule (I)),
caractérisé en ce que
on fait réagir un composé de formule (Iε') 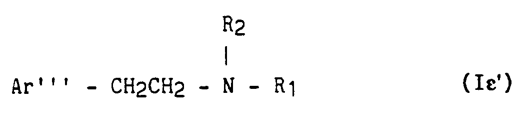
dans laquelle R₁ et R₂ sont tels que définis précédemment et Ar''' représente un groupement Ar' tel que défini dans la formule (I) substitué par un groupement -O-R₃''' avec R₃''' représentant un atome d'hydrogène, avec un composé de formule (XIV)

R'' - Hal (XIV)

dans laquelle Hal représente un atome d'halogène et R'' représente un groupement choisi parmi aryle éventuellement substitué, arylalkyle ou diarylalkyle éventuellement substitués, ou cycloalkyle ou cycloalkylalkyle, (les termes : "aryle", "arylalkyle", "diarylalkyle", "cycloalkyle", "cycloalkylalkyle", et "substitué(s)" étant tels que définis dans la formule (I)),
les composés de formule (Iε) pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

L'invention s'étend également au procédé de préparation des composés de formule (Iφ) : 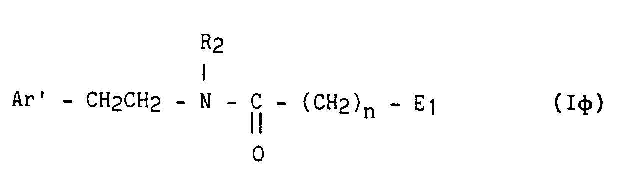
dans laquelle Ar', R₂, E₁, et n sont tels que définis dans la formule (I), caractérisé en ce que on fait réagir un composé de formule (Iφ') 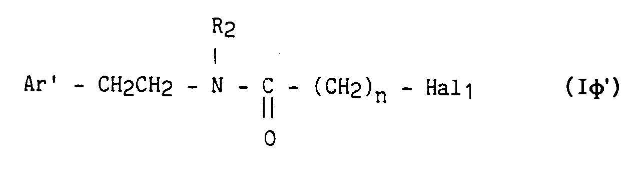
dans laquelle Ar', R₂, et n sont tels que définis dans la formule (I) et Hal₁ représente un atome d'halogène, avec une morpholine ou une pipérazine non substituée ou substituée par un radical -(CH₂)ₙ'-E₂ où n' et E₂ sont tels que définis dans la formule (I),
les composés de formule (Iφ) pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

Les amines de formule **(IX)** sont, soit commerciales, soit aisément accessibles à l'homme de l'art.

Les composés de formule **(I)** possèdent des propriétés pharmacologiques intéressantes.

L'étude pharmacologique de ces composés a en effet montré qu'ils étaient peu toxiques et doués d'une très haute affinité selective pour les récepteurs à la mélatonine (très supérieure à la mélatonine elle même et à ses analogues décrits dans la littérature).

Parmi leurs importantes activités sur le système nerveux central, les composés de l'invention possèdent des propriétés sédatives anxiolytiques, antipsychotiques, analgésiques ainsi que sur la microcirculation qui les rendent utiles dans le traitement du stress, des troubles du sommeil, de l'anxiété, des dépressions saisonnières, des insomnies et fatigues dues aux décalages horaires, des schizophrénies, de l'attaque de panique, de la mélancolie, de la régulation de l'appétit, de l'insomnie, des troubles psychotiques, de l'épilepsie, de la maladie de Parkinson, de la démence sénile, des désordres liés au vieillissement normal ou pathologique, de la migraine, des pertes de mémoire, de la maladie d'Alzheimer ainsi que des troubles de la circulation cérébrale.

Les composés de l'invention possèdent également des propriétés inhibitrices de l'ovulation et d'immunomodulateur les rendant succeptibles d'être utilisés dans le traitement de certains cancers.

Administrés par voie externe, ils sont utiles dans le traitement du psoriasis, de l'acné, de la séborrhée et protègent la peau.

Ils peuvent également avoir un usage vétérinaire pour leurs propriétés sur le pelage.

La présente invention a également pour objet les compositions pharmaceutiques contenant les produits de formule **(I)** seuls ou en combinaison avec un ou plusieurs excipients ou véhicules inertes, non toxiques, pharmaceutiquement acceptables.

Parmi les compositions pharmaceutiques selon l'invention, on pourra citer, à titre d'exemples et de façon non limitative, celles qui conviennent pour l'administration orale, parentérale, nasale, per/ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, les ampoules buvables et injectables.

La posologie varie selon l'âge et le poids du patient, la voie d'administration, la nature de l'indication thérapeutique, ou des traitements éventuellement associés et s'échelonne entre 0,1 mg et 1 gramme par 24 heures.

Les exemples suivants illustrent l'invention, mais ne la limitent en aucune façon.

### EXEMPLE 1 : N-[2-(5-METHOXY INDOL-3-YL) ETHYL] CYCLOPROPYLCARBOXAMIDE

Ajouter 3 g de 5-méthoxy tryptamine à une solution de 2,2 g de carbonate de potassium dans 40 cm³ d'eau. Ajouter 80 cm³ de chloroforme puis ajouter sous très vive agitation 1,7 g de chlorure de l'acide cyclopropane carboxylique. Après 30 minutes d'agitation à température ambiante, décanter la phase organique, la laver à l'eau puis la mettre à sec.

Le résidu obtenu est cristallisé dans du toluène.

On obtient 3,3 g (80,5 %) de N-[2-(5-méthoxy indol-3-yl) éthyl] cyclopropylcarboxamide.
Point de fusion : 101-102° C
Infrarouge (pastille KBr) : 3390 cm⁻¹ ν NH indole
3250-3300 cm⁻¹ ν NH amide
2900-3050 cm⁻¹ ν CH alkyle
1630 cm⁻¹ ν CO amide
RMN ¹H 80 MHz (CDCl₃) 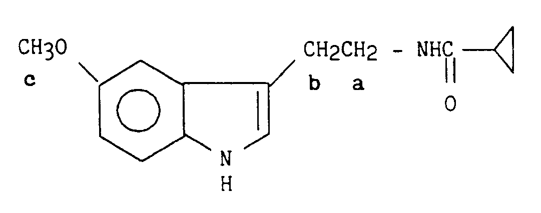
0,7-1 ppm (5H) cyclopropyl
2,8-3 ppm (2H) CH₂ **b**
3,4-3,7 ppm (2H) CH₂ **a**
3,8 ppm (H) OCH₃
6,7-7,3 ppm (4H) indole
8,1 ppm (1H) NH (indole)

### EXEMPLE 2 : N-[2-(6-FLUORO 5-METHOXY INDOL-3-YL) ETHYL] CYCLOPROPYLCARBOXAMIDE

En procédant de la même façon que dans l'exemple 1 mais en remplaçant la 5-méthoxy tryptamine par la 5-méthoxy 6-fluoro tryptamine (J. Heterocyclic Chem. (1976) 13 pp 1253-1256), on obtient la N-[2-(5-méthoxy 6-fluoro indol-3-yl) éthyl] cyclopropylcarboxamide. Point de fusion (dichlorométhane-éther) : 125-126° C

### EXEMPLE 3 : N-[2-(6-CHLORO 5-METHOXY INDOL-3-YL) ETHYL] CYCLOPROPYLCARBOXAMIDE

En procédant de la même façon que dans l'exemple 1 mais en remplaçant la 5-méthoxytryptamine par la 5-méthoxy 6-chloro tryptamine (Synthesis (1983) pp 935-936) on obtient la N-[2-(5-méthoxy 6-chloro indol-3-yl) éthyl] cyclopropylcarboxamide

### EXEMPLE 4 : N-[2-(5-METHOXY 2,6-DIMETHYL INDOL-3-YL) ETHYL] CYCLOPROPYLCARBOXAMIDE

En procédant de la même façon que dans l'exemple 1 mais en remplaçant la 5-méthoxy tryptamine par la 5-méthoxy 2,6-diméthyl tryptamine (Journal of Medicinal Chemistry (1973) 16 pp 757-765) on obtient la N-[2-(5-méthoxy 2,6-dimethyl indol-3-yl) éthyl] cyclopropylcarboxamide.

### EXEMPLE 5 : N-[2-(5-METHOXY BENZO [b] THIOPHEN-3-YL) ETHYL] CYCLOPROPYLCARBOXAMIDE

En procédant de la même façon que dans l'exemple 1 mais en remplaçant la 5-méthoxy tryptamine par le 3-β-aminoéthyl 5-méthoxy benzo [b] thiophène (Journal of Medicinal Chemistry (1970) 13 pp 1205-1208), on obtient le N-[2-(5-méthoxy benzo [b] thiophen-3-yl) éthyl] cyclopropylcarboxamide
Infrarouge (pastille KBr) : 3270 cm⁻¹ ν NH amide
1640 cm⁻¹ ν CO amide
Point de fusion : 124-126° C

### EXEMPLE 6 : N-[2-(6-CHLORO 5-METHOXY BENZO [b] THIOPHEN-3-YL) ETHYL] CYCLOPROPYLCARBOXAMIDE

En procédant de la même façon que dans l'exemple 1 mais en remplaçant la 5-méthoxy tryptamine par le 3-β-aminoéthyl 5-méthoxy 6-chloro benzo [b] thiophène (J. Heterocyclic Chem. (1983) 20 pp 1671-1703) on obtient le N-[2-(5-méthoxy 6-chloro benzo [b] thiophen-3-yl) éthyl] cyclopropylcarboxamide.

### EXEMPLE 7 : N-[2-(5-METHOXY BENZO [b] FURAN-3-YL) ETHYL] CYCLOPROPYLCARBOXAMIDE

En procédant de la même façon que dans l'exemple 1 mais en remplaçant la 5-méthoxy tryptamine par le 3-β-aminoéthyl 5-méthoxy benzo [b] furane (Annalen (1963) 662 pp 147-159 ou Aust. J. Chem. (1975) 28 pp 1097-1111) on obtient le N-[2-(5-méthoxy benzo [b] furan-3-yl) éthyl] cyclopropylcarboxamide.
Infrarouge (pastille KBr) : 3290 cm⁻¹ ν NH amide
1680 cm⁻¹ ν C=O amide
RMN ¹H 80 MHz (CDCl₃) 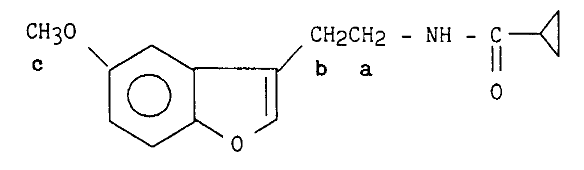
0,7-1 ppm (5H) cyclopropyl
2,9 ppm (2H) CH₂ **b**
3,5-3,7 ppm (2H) CH₂ **a**
3,9 ppm (3H) OCH₃
6,8-7,3 ppm (4H) benzo [b] furane

### EXEMPLE 8 : N-[2-(2-METHYL 5-METHOXY BENZO [b] FURAN-3-YL) ETHYL] CYCLOPROPYLCARBOXAMIDE

En procédant de la même façon que dans l'exemple 1 mais en remplaçant la 5-méthoxy tryptamine par le 2-méthyl 3-β-aminoéthyl 5-méthoxy benzo [b] furane (Brevet FR 1343073) on obtient le N-[2-(2-méthyl 5-méthoxy benzo [b] furan-3-yl) éthyl] cyclopropylcarboxamide.
Infrarouge (pastille KBr) : 3320 cm⁻¹ ν NH amide
1650 cm⁻¹ ν CO amide

### EXEMPLE 9 : N-[2-(6-METHOXY BENZIMIDAZOL-1-YL) ETHYL] CYCLOPROPYLCARBOXAMIDE

En procédant de la même façon que dans l'exemple 1 mais en remplaçant la 5-méthoxy tryptamine par le 1-β-aminoéthyl 6-méthoxy benzimidazole (J. Chem. Soc (1957) pp 1671-1674) on obtient le N-[2-(6-méthoxy benzimidazol-1-yl) éthyl] cyclopropylcarboxamide.
Infrarouge (pastille KBr) : 3300 cm⁻¹ ν NH amide
1660 cm⁻¹ ν CO amide
Point de fusion (ethyl acétate) : 86-88° C

### EXEMPLE 10 : N-[2-(2-BENZYL 6-METHOXY BENZIMIDAZOL-1-YL) ETHYL] CYCLOPROPYLCARBOXAMIDE

En procédant de la même façon que dans l'exemple 1 mais en remplaçant la 5-méthoxy tryptamine par le 1-β-aminoéthyl 2-benzyl 6-méthoxy benzimidazole (Brevet FR 2182915) on obtient le N-[2-(2-benzyl 6-méthoxy benzimidazol-1-yl) éthyl] cyclopropylcarboxamide.

### EXEMPLE 11 : N-[2-(5-METHOXY 1,2-BENZISOXAZOL-3-YL) ETHYL] CYCLOPROPYLCARBOXAMIDE

En procédant de la même façon que dans l'exemple 1 mais en remplaçant la 5-méthoxy tryptamine par le 3-β-aminoéthyl 5-méthoxy 1,2-benzisoxazole (Chem. Pharm. Bull (1976) 24 (4) pp 632-643) on obtient le N-[2-(5-méthoxy 1,2-benzisoxasol-3-yl) éthyl] cyclopropylcarboxamide.

### EXEMPLE 12 : N-[2-(5-METHOXY 1,2-INDAZOL-3-YL) ETHYL] CYCLOPROPYLCARBOXAMIDE

En procédant de la même façon que dans l'exemple 1 mais en remplaçant la 5-méthoxy tryptamine par le 3-β-aminoéthyl 5-méthoxy indazole (J.A.C.S (1957) 79 PP 5245-7) on obtient le N-[2-(5-méthoxy 1,2-indazol-3-yl) éthyl] cyclopropylcarboxamide.

### EXEMPLE 13 : N-[2-(5-METHOXY INDOL-3-YL) ETHYL] TRIFLUOROACETAMIDE

Ajouter goutte à goutte 1,14 g d'acide trifluoroacétique à une suspension à - 5° C de 1,90 g de 5-méthoxy tryptamine dans 6 cm³ de pyridine. Agiter 30 minutes à température ambiante puis verser le milieu réactionnel sur de l'eau glacée. Le précipité formé est isolé par filtration, lavé à l'eau, séché puis recristallisé dans du toluène.

On obtient 1,14 g (40 %) de N-[2-(5-méthoxy indol-3-yl) éthyl] trifluoroacetamide.
Point de fusion : 135-136° C
Infrarouge (pastille KBr) : 3400 cm⁻¹ ν NH indole
3300 cm⁻¹ ν NH amide
1700 cm⁻¹ ν C = O
RMN ¹H 80 MHz (CDCl₃) 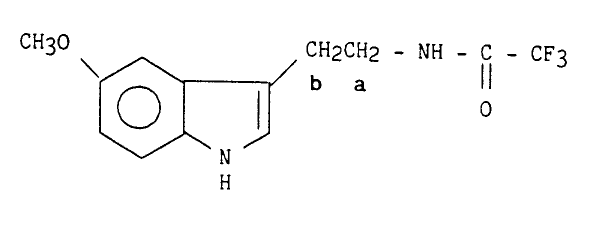
3 ppm (2H) CH₂ **b**
3,6 ppm (2H) CH₂ **a**
3,8 ppm (3H) OCH₃
6,8-7,3 ppm protons aromatiques

### EXEMPLE 14 : N-[2-(5-METHOXY BENZO [b] THIOPHEN-3-YL) ETHYL] TRIFLUOROACETAMIDE

En procédant de la même façon que dans l'exemple 13 mais en remplaçant la 5-méthoxy tryptamine par le 3-β-aminoéthyl 5-méthoxy benzo [b] thiophène on obtient le N-[2-(5-méthoxy benzo [b] thiophen-3-yl) éthyl] trifluoroacétamide.
Infrarouge (pastille KBr) : 3280 cm⁻¹ ν NH amide
1690 cm⁻¹ ν C = O

### EXEMPLE 15 : N-[2-(5-METHOXY BENZO [b] FURAN-3-YL) ETHYL] TRIFLUOROACETAMIDE

En procédant de la même façon que dans l'exemple 13 mais en remplaçant la 5-méthoxy tryptamine par le 3-β-aminoéthyl 5-méthoxy benzo [b] furane on obtient le N-[2-(5-méthoxy benzo [b] furan-3-yl) éthyl] trifluoroacétamide.
Infrarouge (pastille KBr) : 3290 cm⁻¹ ν NH amide
1700 cm⁻¹ ν C = O amide

### EXEMPLE 16 : N-[2-(6-METHOXY BENZIMIDAZOL-1-YL) ETHYL] TRIFLUOROACETAMIDE

En procédant de la même façon que dans l'exemple 13 mais en remplaçant la 5-méthoxy tryptamine par le 1-β-aminoéthyl 6-méthoxy benzimidazole on obtient le N-[2-(6-méthoxy benzimidazol-1-yl) éthyl] trifluoroacetamide.
Infrarouge (pastille KBr) : 3300 cm⁻¹ ν NH amide
1690 cm⁻¹ ν C = O amide

### EXEMPLE 17 : N-[2-(5-METHOXY 1,2-BENZISOXAZOL-3-YL) ETHYL] TRIFLUROACETAMIDE

En procédant de la même façon que dans l'exemple 13 mais en remplaçant la 5-méthoxy tryptamine par le 3-β-aminoéthyl 5-méthoxy 1,2-benzisoxazole on obtient le N-[2-(5-méthoxy 1,2-benzisoxazol-3-yl) éthyl] trifluoroacétamide.

### EXEMPLE 18 : N-[2-(5-METHOXY INDOL-3-YL) ETHYL] N'-PROPYL UREE

Ajouter 0,851 g d'isocyanate de propyle à une suspension de 1,902 g de 5-méthoxy tryptamine dans 4 cm³ de pyridine à + 5°C. Agiter 2 heures à température ambiante puis verser le milieu réactionnel sur de l'eau glacée. Acidifier légèrement avec une solution d'acide chlorhydrique 1N. Le précipité formé est isolé par filtration, lavé à l'eau, séché puis recristallisé dans du toluène, on obtient 2,34 g (85 %) de N-[2-(5-méthoxy indol-3-yl) éthyl] N'-propyl urée.
Point de fusion : 79-80° C
RMN ¹H 80 MH_{z} (CDCl₃) 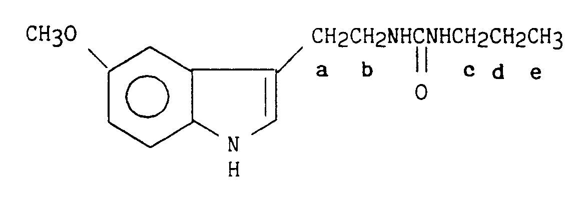
0,9 ppm (3H) CH₃ **e**
1,4 ppm (2H) CH₂ **d**
2,9 ppm (4H) CH₂ **a** CH₂ **c**
3,4 ppm (2H) CH₂ **b**
3,9 ppm (3H) OCH₃
6,6-7,3 ppm protons aromatiques

### EXEMPLE 19 : N-[2-(5-METHOXY BENZO [b] THIOPHEN-3-YL) ETHYL] N'-PROPYL UREE

En procédant de la même façon que dans l'exemple 18 mais en remplaçant la 5-méthoxy tryptamine par le 3-β-aminoéthyl 5-méthoxy benzo [b] thiophène, on obtient la N-[2-(5-méthoxy benzo [b] thiophèn-3-yl) éthyl] N'-propyl urée.
Infrarouge (pastille KBr) : 3300 cm⁻¹ ν NH
1620 cm⁻¹ ν C = O

### EXEMPLE 20 : N-[2-(6-CHLORO 5-METHOXY INDOL-3-YL) ETHYL] N'-PROPYL UREE

En procédant de la même façon que dans l'exemple 18 mais en remplaçant la 5-méthoxy tryptamine par la 5-méthoxy 6-chloro tryptamine, on obtient la N-[2-(5-méthoxy 6-chloro indol-3-yl)éthyl] N'-propyl urée
Infrarouge (pastille KBr) : 3250 cm⁻¹ ν NH
1620 cm⁻¹ ν C = O
RMN ¹H 80 MH_{z} (CDCl₃) 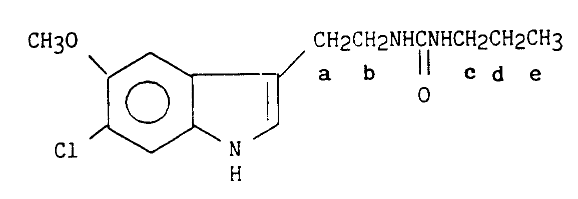
0,9-1 ppm (3H) CH₃ **e**
1,5 ppm (2H) CH₂ **d**
2,8-3 ppm (4H) CH₂ **b** CH₂ **c**
3,4 ppm (2H) CH₂ **a**
3,90 ppm (3H) OCH₃

### EXEMPLE 21 : N-[2-(5-METHOXY BENZO [b] FURAN-3-YL) ETHYL] N'-PROPYL UREE

En procédant de la même façon que dans l'exemple 18 mais en remplçant la 5-méthoxy tryptamine par le 3-β-aminoéthyl 5-méthoxy benzo [b] furane, on obtient la N-[2-(5-méthoxy benzo [b] furan-3-yl) éthyl] N'-propyl urée
Infrarouge (pastille KBr) : 3290 cm⁻¹ ν NH
1620 cm⁻¹ ν C = O

### EXEMPLE 22 : N-[2-(5-METHOXY INDOL-3-YL) ETHYL] N'-PROPYL THIOUREE

Ajouter 1,11 g d'isothiocyanate de propyle à une suspension de 2,27 g de 5-méthoxy tryptamine dans 5 cm3 de pyridine.

Le milieu réactionnel est agité 1 heure à 80° puis, après refroidissement, versé sur un mélange d'eau et de glace et acidifié légèrement avec une solution d'acide chlorhydrique 1N.

Le précipité formé est isolé par filtration , lavé à l'eau, séché puis recristallisé dans du toluène.

On obtient ainsi 2,18 g (75 %) de N-[2-(5-méthoxy indol-3-yl) éthyl] N'-propyl thiourée
RMN ¹H 80 MH_{z} (CDCl₃) 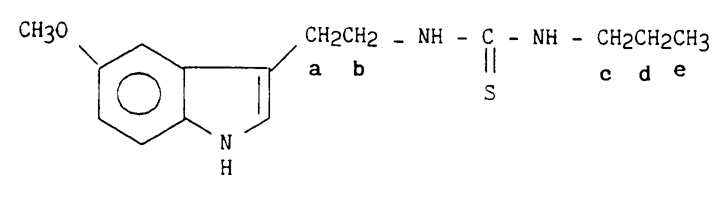
0,85 ppm (3H) CH₃ **e**
1,45 ppm (2H) CH₂ **d**
2,95 ppm (4H) CH₂ **c** CH₂ **a**
3,4 ppm (2H) CH₂ **b**
3,85 ppm (3H) OCH₃
5,50 ppm (2H) 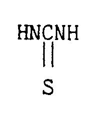
disparait dans D₂O
6,7-7,3 ppm protons aromatiques

### EXEMPLE 23 : N-[2-(5-METHOXY INDOL-3-YL) ETHYL] CYCLOBUTYLCARBOXAMIDE

En procédant comme dans l'exemple 1, mais en remplaçant le chlorure de l'acide cyclopropane carboxylique par le chlorure de l'acide cyclobutane carboxylique, on obtient le composé du titre
Point de fusion : 111 - 112°C
Solvant de cristallisation : chloroforme-acétone

### EXEMPLES 24 A 25 :

En procédant comme dans l'exemple 1, mais en remplaçant le chlorure de l'acide cyclopropane carboxylique par le chlorure d'acide approprié, ou son anhydride d'acide correspondant le cas échéant, on obtient les composés des exemples suivants :

### EXEMPLE 24 : N-[2-(5-METHOXY INDOL-3-YL) ETHYL] CYCLOHEXYLCARBOXAMIDE

### EXEMPLE 25 : N-[2-(5-METHOXY INDOL-3-YL) ETHYL] 3-CYCLOPENTYLPROPIONAMIDE

### EXEMPLE 26 : N-[2-(5-METHOXY INDOL-3-YL) ETHYL] MORPHOLINOACETAMIDE

### Stade I : N-[2-(5-METHOXY INDOL-3-YL) ETHYL] BROMOACETAMIDE

En procédant comme dans l'exemple 1 mais en remplaçant le chlorure de l'acide cyclopropane carboxylique par le chlorure de l'acide bromoacétique on obtient le N-[2-(5-méthoxy indol-3-yl) éthyl] bromoacétamide

### Stade II : N-[2-(5-METHOXY INDOL-3-YL) ETHYL] MORPHOLINO ACETAMIDE

Dissoudre sous agitation magnétique 0,01 mole de morpholine dans 50 cm³ d'acétone. Ajouter 0,012 mole de triéthylamine et 0,01 mole de N-[2-(5-méthoxy indol-3-yl) éthyl] 2-bromcacétamide. Porter à reflux 1 heure sous agitation magnétique. Essorer le précipité formé et évaporer le filtrat.
Reprendre le résidu par de l'eau alcaline essorer le précipité, laver, sécher et recristalliser dans un mélange toluène-cyclohexane pour obtenir le composé du titre.

### EXEMPLES 27 ET 28 :

En procédant comme dans l'exemple 26, mais en remplaçant au stade II la morpholine par la 1-benzylpipérazine puis par la 1-(2,3,4-triméthoxybenzyl)pipérazine, on obtient successivement les composés des exemples suivants :

### EXEMPLE 27 : N-[2-(5-METHOXY INDOL-3-YL) ETHYL] 2-(4-BENZYLPIPERAZIN-1-YL) ACETAMIDE

### EXEMPLE 28 : N-[2-(5-METHOXY INDOL-3-YL) ETHYL] 2-(4-(2,3,4-TRIMETHOXYBENZYL)PIPERAZIN-1-YL) ACETAMIDE

### EXEMPLE 29 : N-[2-(5-HYDROXY INDOL-3-YL) ETHYL] CYCLOPROPYLCARBOXAMIDE

En procédant comme dans l'exemple 1, mais en remplaçant la 5-méthoxy tryptamine par la 5-hydroxy tryptamine on obtient le composé du titre

### EXEMPLE 30 : N-{2-{5-[(CYCLOHEXEN-3-YL)OXY] INDOL-3-YL} ETHYL} CYCLOPROPYLCARBOXAMIDE

Dans un ballon rodé de 50 cm³, introduire 1,98.10⁻² mole de carbonate de potassium, 1,33.10⁻² mole de N-[2-(5-hydroxy indol-3-yl) éthyl] cyclopropylcarboxamide dissout dans 20 cm³ d'acétone anhydre, et 2,1.10⁻² mole de 3-bromocyclohexène. Chauffer à reflux pendant 22 heures. Le milieu réactionnel est filtré et le filtrat évaporé sous pression réduite. La recristallisation du résidu d'évaporation dans l'acétate d'éthyle permet d'obtenir le N-{2-{5-[(cyclohexène-3-yl)oxy] indol-3-yl} ethyl} cyclopropylcarboxamide purifié.

### EXEMPLE 31 : N-[2-(5-BENZYLOXY INDOL-3-YL) ETHYL] CYCLOPROPYLCARBOXAMIDE

Dans un ballon de 150 cm³ contenant 50 cm³ d'éthanol absolu, ajouter sous agitation magnétique et par petites fractions 0,23 gramme de sodium.

Ajouter ensuite 0,01 mole de N-[2-(5-hydroxy indol-3-yl) éthyl] cyclopropylcarboxamide, poursuivre l'agitation pendant 30 mn, puis évaporer à sec.

Le dérivé sodé obtenu est dissous dans 30 cm³ de diméthyl formamide anhydre. Sous agitation magnétique, ajouter 0,011 mole de bromure de benzyle par l'intermédiaire d'une ampoule à brome.

Chauffer à 90°C pendant 4 heures. Laisser refroidir, puis reverser le milieu réactionnel sur de la glace. Essorer la précipité formé, laver avec une solution d'hydroxyde de sodium 1N puis à l'eau. Sécher et recristalliser pour obtenir le N-[2-(5-benzyloxy indol-3-yl) éthyl] cyclopropylcarboxamide purifié.

### EXEMPLES 32 A 37

En procédant comme dans l'exemple 18, mais en remplaçant l'isocyanate de propyle par les isocyanates ou isothiocyanates appropriés, on obtient les composés des exemples suivants :

### EXEMPLE 32 : N-[2-(5-METHOXY INDOL-3-YL) ETHYL] N'-BENZYL UREE

### EXEMPLE 33 : N-[2-(5-METHOXY INDOL-3-YL) ETHYL] N'-CYCLOPROPYL UREE

### EXEMPLE 34 : N-[2-(5-METHOXY INDOL-3-YL) ETHYL] N'-CYCLOBUTYL UREE

### EXEMPLE 35 : N-[2-(5-METHOXY INDOL-3-YL) ETHYL] N'-BUTYL UREE

### EXEMPLE 36 : N-[2-(5-METHOXY INDOL-3-YL) ETHYL] N'-PROPYLTHIOUREE

### EXEMPLE 37 : N-[2-(5-METHOXY INDOL-3-YL) ETHYL] N'-CYCLOHEXYL THIOUREE

### EXEMPLES 38 ET 39

En procédant comme dans l'exemple 5 mais en remplaçant le chlorure de l'acide cyclopropane carboxylique par le chlorure d'acide ou l'anhydride d'acide appropriés, on obtient les composés des exemples suivants :

### EXEMPLE 38 : N-[2-(5-METHOXY BENZO [b] THIOPHEN-3-YL) ETHYL] CYCLOBUTYLCARBOXAMIDE

### EXEMPLE 39 : N-[2-(5-METHOXY BENZO [b] THIOPHEN-3-YL) ETHYL] CYCLOOCTYLCARBOXAMIDE

### EXEMPLES 40 ET 41

En procédant comme dans l'exemple 19 mais en remplaçant l'isocyanate de propyle par l'isocyanate ou isothiocyanate approprié, on obtient les composés des exemples suivants :

### EXEMPLE 40 : N-[2-(5-METHOXY BENZO [b] THIOPHEN-3-YL) ETHYL] N'-CYCLOPROPYLUREE

### EXEMPLE 41 : N-[2-(5-METHOXY BENZO [b] THIOPHEN-3-YL) ETHYL] N'-CYCLOHEXYLTHIOUREE

### EXEMPLES 42 ET 43

En procédant comme dans l'exemple 6 mais en remplaçant le chlorure de l'acide cyclopropane carboxylique par le chlorure d'acide approprié, on obtient les composés des exemples suivants :

### EXEMPLE 42 : N-[2-(6-CHLORO 5-METHOXY BENZO [b] THIOPHEN-3-YL) ETHYL] CYCLOBUTYLCARBOXAMIDE

### EXEMPLE 43 : N-[2-(6-CHLORO 5-METHOXY BENZO [b] THIOPHEN-3-YL) ETHYL] 3-CYCLOPENTYLPROPIONAMIDE

### EXEMPLES 44 A 46

En procédant comme dans l'exemple 7 mais en remplaçant le chlorure de l'acide cyclopropane carboxylique par les chlorures d'acide appropriés, on obtient les composés des exemples suivants :

### EXEMPLE 44 : N-[2-(BENZO [b] FURAN-3-YL) ETHYL] CYCLOBUTYLCARBOXAMIDE

### EXEMPLE 45 : N-[2-(BENZO [b] FURAN-3-YL) ETHYL] CYCLOHEXYLCARBOXAMIDE

### EXEMPLE 46 : N-[2-(BENZO [b] FURAN-3-YL) ETHYL] TRIFLUOROACETAMIDE

### EXEMPLES 47 A 51

En procédant comme dans l'exemple 21 mais en remplaçant l'isocyanate de propyle par l'isocyanate ou l'isothiocyanate approprié, on obtient les composés des exemples suivants :

### EXEMPLE 47 : N-[2-(5-METHOXY BENZO [b] FURAN-3-YL) ETHYL] N'-METHYLUREE

### EXEMPLE 48 : N-[2-(5-METHOXY BENZO [b] FURAN-3-YL) ETHYL] N'-ETHYLUREE

### EXEMPLE 49 : N-[2-(5-METHOXY BENZO [b] FURAN-3-YL) ETHYL] N'-HEXYLUREE

### EXEMPLE 50 : N-[2-(5-METHOXY BENZO [b] FURAN-3-YL) ETHYL] N'-BENZYLUREE

### EXEMPLE 51 : N-[2-(5-METHOXY BENZO [b] FURAN-3-YL) ETHYL] N'-PROPYLTHIOUREE

### EXEMPLES 52 A 54

En procédant comme dans l'exemple 11 mais en remplaçant le chlorure de l'acide cyclopropane carboxylique par le chlorure de l'acide approprié, on obtient les composés des exemples suivants :

### EXEMPLE 52 : N-[2-(5-METHOXY 1,2-BENZISOXAZOL-3-YL) ETHYL] ACETAMIDE

### EXEMPLE 53 : N-[2-(5-METHOXY 1,2-BENZISOXAZOL-3-YL) ETHYL] BUTYRAMIDE

### EXEMPLE 54 : N-[2-(5-METHOXY 1,2-BENZISOXAZOL-3-YL) ETHYL] 3-CHLOROPROPIONAMIDE

### EXEMPLES 55 ET 56

En procédant comme dans l'exemple 12 mais en remplaçant le chlorure de l'acide cyclopropane carboxylique par le chlorure de l'acide approprié, on obtient les composés des exemples suivants :

### EXEMPLE 55 : N-[2-(5-METHOXY 1,2-INDAZOL-3-YL) ETHYL] ACETAMIDE

### EXEMPLE 56 : N-[2-(5-METHOXY 1,2-INDAZOL-3-YL) ETHYL] PROPIONAMIDE

### EXEMPLES 57 A 60

En procédant comme dans l'exemple 9, mais en remplaçant le chlorure de l'acide cyclopropane carboxylique par le chlorure de l'acide correspondant, on obtient les composés des exemples suivants :

### EXEMPLE 57 : N-[2-(6-METHOXY BENZIMIDAZOL-1-YL) ETHYL] ACETAMIDE

Point de fusion : 173 - 175°C

### EXEMPLE 58 : N-[2-(6-METHOXY BENZIMIDAZOL-1-YL) ETHYL] BUTYRAMIDE

### EXEMPLE 59 : N-[2-(6-METHOXY BENZIMIDAZOL-1-YL) ETHYL] PENTANAMIDE

### EXEMPLE 60 : N-[2-(6-METHOXY BENZIMIDAZOL-1-YL) ETHYL] 2-BROMOACETAMIDE

### EXEMPLES 61 A 64

En procédant de la même façon que dans l'exemple 16 mais en remplaçant l'isocyanate de propyle par les isocyanates ou isothiocyanates appropriés, on obtient les composés des exemples suivants :

### EXEMPLE 61 : N-[2-(6-METHOXY BENZIMIDAZOL-1-YL) ETHYL] N'-PROPYLUREE

### EXEMPLE 62 : N-[2-(6-METHOXY BENZIMIDAZOL-1-YL) ETHYL] N'-BENZYLUREE

### EXEMPLE 63 : N-[2-(6-METHOXY BENZIMIDAZOL-1-YL) ETHYL] N'-PROPYLTHIOUREE

### EXEMPLE 64 : N-[2-(6-METHOXY BENZIMIDAZOL-1-YL) ETHYL] N'-CYCLOHEXYLTHIOUREE

### EXEMPLE 65 : N-[2-(6-FLUORO 5-METHOXYINDOL-3-YL) ETHYL] N'-PROPYLUREE

Point de fusion (dichlorométhane-éther) : 109-110° C

### EXEMPLE A : DETERMINATION DE LA LIAISON AUX RECEPTEURS DE LA MELATONINE

La liaison aux récepteurs de la mélatonine des composés de l'invention a été réalisée selon des techniques classiques sur des récepteurs de la Pars Tuberalis de moutons (Journal of Neuroendocrinology Vol 1 N° 1 pages 1 à 4 (1989)).

Les composés de l'invention se lient de manière extrêmement spécifiques aux récepteurs de la mélatonine avec, pour les plus affins d'entre eux, une affinité de plus de 100 fois supérieure à celle de la mélatonine elle-même. Les composés de l'invention testés ont une constante de dissociation (Kd) de l'ordre de 10-¹³ mol. l-¹ contre 6,3.10-¹¹ mol.1-¹ pour la mélatonine elle-même.

### EXEMPLE B : TEST DES QUATRE PLAQUES

Les produits de l'invention sont administrés par voie oesophagienne à des lots de dix souris. Un lot reçoit du sirop de gomme.
30 minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre des passages est enregistré pendant une minute. Après administration, les composés de l'invention augmentent de façon significative le nombre de passages ce qui montre l'activité anxiolytique ces dérivés de l'invention.

### EXEMPLE C : ACTIVITE DES PRODUITS DE L'INVENTION SUR LA MICROCIRCULATION ISCHEMIQUE

L'étude expérimentale a été réalisée sur les muscles crémasters de rats mâles (Sprague-Dawley) après ligature de l'artère iliaque commune.

Les muscles ont été placés dans une chambre transparente, perfusés par une solution de tampon bicarbonate équilibrée par un mélange gazeux CO2/N2 5/95%. La vélocité des globules rouges et le diamètre des artérioles de premier ou second ordre irriguant le crémaster ont été mesurés, le flux sanguin artériolaire a été calculé. Des informations identiques ont été obtenues pour quatre types de vaisseaux.

On a effectué le même type de mesure simultanément :
- sur le crémaster perfusé normalement,
- sur le crémaster sous ligature, c'est-à-dire le crémaster ischémié 2, 7, 14 et 21 jours après ligature.

Deux groupes d'animaux ont été étudiés :
- un groupe témoin sans traitement,
- un groupe traité per os par un produit de l'invention, à raison de 0,1 mg.kg-1 par jour.

On n'a constaté aucune différence ni dans la vélocité des globules ni dans le diamètre des vaisseaux dans les muscles crémasters normalement irrigués chez les animaux traités par rapport aux témoins.

Par contre, au niveau du muscle crémaster ischémié, le diamètre moyen des artérioles était amélioré chez les animaux traités par rapport aux témoins. La vélocité des globules rouges était normalisée par un traitement de 21 jours.

En fait, chez les animaux traités, la vélocité des globules rouges et le débit sanguin mesurés 7 jours après la ligature, ne présentent pas de différence significative avec les valeurs obtenues dans le crémaster non ischémié. Ces résultats sont obtenus sans modification de la pression artérielle.

Ces résultats indiquent que le traitement chronique par l'un des composés de l'invention améliore la microcirculation et l'irrigation sanguine des territoires ischémiés.

### EXEMPLE D : STIMULATION DES REPONSES IMMUNITAIRES

A des groupes de six souris, on a administré des globules rouges de moutons. Ces groupes de souris ont ensuite été traités par voie sous-cutanée par les composés de l'invention pendant six jours et un groupe témoin a été traité par un placébo. Les souris sont ensuite laissées au repos pendant quatre semaines puis ont ensuite reçu une injection de rappel de globules rouges de mouton sans reçevoir de nouvelles administrations de produit de l'invention. La réponse immunitaire a été évaluée 3 jours après l'injection de rappel. Elle est statistiquement accrue dans le groupe traité par les composés de l'invention.

### EXEMPLE E : INHIBITION DE L'OVULATION

On utilise des rats femelles adultes avec des cycles réguliers de quatre jours.

Des frottis vaginaux quotidiens ont été réalisés et des rates ont été sélectionnées après qu'elles aient montré au moins deux cycles consécutifs de quatre jours.

Chaque cycle est constitué de deux jours de dioestrus, un jour de proestrus et un jour d'oestrus.

L'après-midi du jour de proestrus, l'hormone lutéinisante est libérée dans le sang par l'hypophyse. Cette hormorne induit l'ovulation qui se traduit par la présence d'oeufs au niveau de l'oviducte le jour de l'oestrus.

Les composés de l'invention sont administrés par voie orale à midi le jour de l'oestrus. Les rates traitées et témoins sont sacrifiées le jour de l'oestrus. Les oviductes sont examinés. On remarque un pourcentage significatif de diminution du nombre des oeufs dans les oviductes de rates traitées par les composés de l'invention.

### EXEMPLE F : COMPOSITION PHARMACEUTIQUE : COMPRIMES comprimés dosés a 5 mg de N-[2-(5-METHOXY INDOL-3-YL) ETHYL] N'-PROPYL UREE

Formule de préparation pour 1000 comprimés :

| | |
|---|---|
| N-[2-(5-méthoxy indol-3-yl) éthyl] N'-propyl urée | 5 g |
| Amidon de blé | 20 g |
| Amidon de maïs | 20 g |
| Lactose | 30 g |
| Stéarate de magnésium | 2 g |
| Silice | 1 g |
| Hydroxypropyl cellulose | 2 g |

## Revendications (Revendications pour l'(les) Etat(s) contractant(s) suivant(s): AT, BE, CH, DE, DK, FR, GB, IE, IT, LI, LU, NL, PT, SE)

1. Composés de formule générale **(I)** : 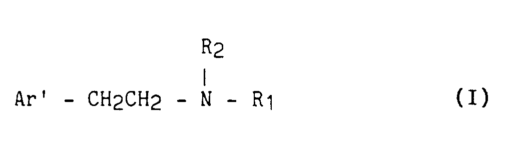 dans laquelle :
- Ar' représente :
- un noyau indol-3-yl de formule **(II)** : 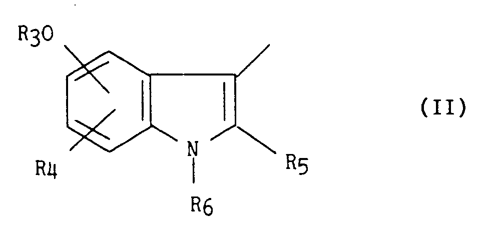
- un noyau benzo [b] thiophèn-3-yl de formule **(III)** : 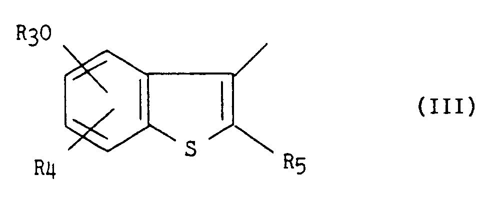
- un noyau benzimidazol-1-yl de formule **(IV)** : 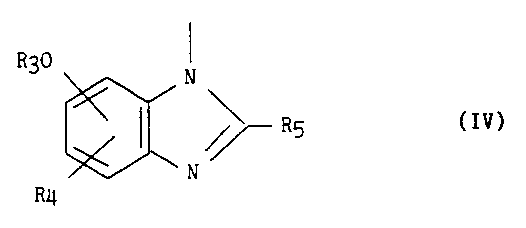
- un noyau benzo [b] furan-3-yl de formule **(V)** : 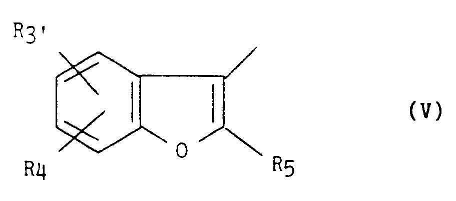
- un noyau 1,2-benzisoxazol-3-yl de formule **(VI)** : 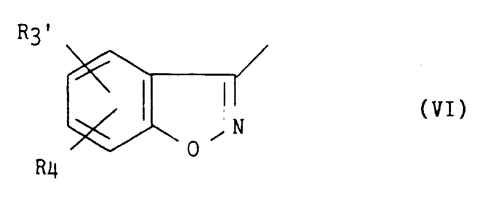
- un noyau 1,2-benzisothiazol-3-yl de formule **(VII)** : 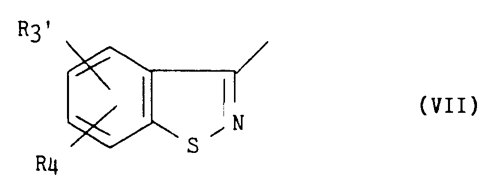
- un noyau indazol-3-yl de formule **(VIII)** : 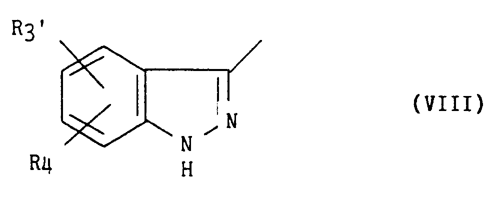
- R₁ représente :
- un groupement 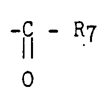 dans lequel R₇ représente un cycloalkyle éventuellement substitué, un cycloalkyl-(C₁-C₄)alkyle éventuellement substitué, ou un trifluorométhyle,
étant entendu que lorsque Ar' représente un groupement choisi parmi ceux de formule (IV), (VI), (VII), et (VIII), alors R₇ peut également représenter un alkyle linéaire ou ramifié de 1 à 6 atomes de carbone non substitué ou substitué par 1 à 2 radicaux halogène,
- un groupement 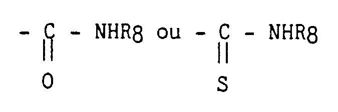 dans lesquels R₈ représente un alkyle inférieur de 1 à 6 atomes de carbone linéaire ou ramifié, un cycloalkyle éventuellement substitué, un cycloalkyl-(C₁-C₄)alkyle éventuellement substitué, un aryle éventuellement substitué, un arylalkyle éventuellement substitué dont la chaîne alkyle comporte de 1 à 3 atomes de carbone,
- un groupement 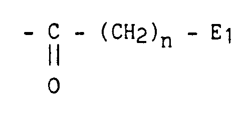 dans lequel
n représente un nombre entier compris entre 1 et 3, et E₁ représente un radical choisi parmi :
- morpholino,
- pipérazine non substituée ou substituée par un radical -(CH₂)ₙ'-E₂ où n' représente un entier de 1 à 4 et E₂ représente un radical phényl ou naphtyl non substitué ou substitué par un a trois radicaux choisis parmi : halogène, (C₁-C₄) alkyl, et (C₁-C₄) alcoxy,
- R₂ représente un hydrogène ou un alkyle inférieur de 1 à 6 atomes de carbone linéaire ou ramifié,
- R₃ représente un hydrogène, un alkyle inférieur de 1 à 6 atomes de carbone linéaire ou ramifié, un aryle éventuellement substitué, un arylalkyle ou diarylalkyle éventuellement substitués dans lesquels la chaîne alkyle comporte de 1 à 3 atomes de carbone, ou un cycloalkyle ou un cycloalkylalkyle dans lequel la chaîne alkyle comporte de 1 à 3 atomes de carbone,
- R₃' représente un atome d'hydrogène ou un groupement -O-R₃ avec R₃ tel que défini précédemment,
- R₄ représente un hydrogène, un halogène, un hydroxy, un alcoxy de 1 à 6 atomes de carbone linéaire ou ramifié, un alkyle inférieur de 1 à 6 atomes de carbone linéaire ou ramifié,
- R₅ représente un hydrogène, un halogène, un alkyle inférieur de 1 à 6 atomes de carbone linéaire ou ramifié, un phényle éventuellement substitué, un phénylalkyle dont la chaîne alkyle comprend de 1 à 3 atomes de carbone, éventuellement substitué,
- R₆ représente un hydrogène, un alkyle inférieur de 1 à 6 atomes de carbone linéaire ou ramifié,
- leurs isomères, épimères, diastéréoisomères,
- et leurs sels d'addition à un acide ou à une base pharmaceutiquement acceptable,
- avec les réserves que :
· Ar' ne peut pas représenter un groupement 7-méthoxy benzo [b] furan-3-yl lorsque R₁ représente un cyclopropylcarbonyle,
· R₁ ne peut pas représenter un trifluoroacétyle, lorsque Ar' représente un indole avec R₂ = R₃ = R₄ = R₅ = R₆ = H,
· R₁ ne peut pas représenter un radical anilinothiocarbonyl non substitué ou substitué en position 4 du phényl par un radical alcoxy lorsque Ar' représente un noyau indol-3-yl et R₃ représente un radical méthyle ou benzyle,
· et R₁ ne peut pas représenter un groupement -CO-NH-R₈ lorsque Ar' représente un noyau de formule (II) et R₅ représente un phénylalkyle dont la chaîne alkyle comprend de 1 à 3 atomes de carbone, éventuellement substitué,
étant entendu que, sauf précisions contraires :
- le terme "substitué" associé aux expressions "aryle", "arylalkyle", diarylalkyle", phényle", et "phénylalkyle" signifie que le ou les noyaux aromatiques peuvent être substitués par un ou plusieurs radicaux choisis parmi : alkyle inférieur de 1 à 6 atomes de carbone, linéaire ou ramifié, alcoxy inférieur de 1 à 6 atomes de carbone, linéaire ou ramifié, hydroxy, halogène, nitro, et trifluorométhyle,
- le terme "substitué" associé aux expressions "cycloalkyle", et "cycloalkyl-(C₁-C₄)lalkyle" signifie que le système cyclique peut être substitué par un ou plusieurs radicaux choisis parmi : halogène, alkyle inférieur de 1 à 6 atomes de carbone, linéaire ou ramifié, et alcoxy inférieur de 1 à 6 atomes de carbone, linéaire ou ramifié,
- le terme "cycloalkyle" désigne un système cyclique, saturé ou insaturé, de 3 à 8 atomes de carbone,
- par groupement aryle on entend groupement pyridyle, phényle, naphtyle, thiényle, furyle, ou pyrimidyle.

2. Composés selon la revendication 1 pour lesquels Ar' représente un noyau indol-3-yl ce qui correspond aux indoles de formule : 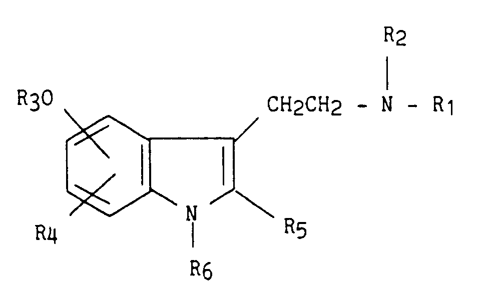 dans laquelle R₁, R₂, R₃, R₄, R₅, et R6 ont la même signification que dans la revendication 1,
leurs isomères, épimères, et diastéréoisomères, ainsi que leurs sels d'addition à un acide ou à une base pharmaceutiquement acceptable.

3. Composés selon la revendication 1 pour lesquels Ar' représente un noyau benzo [b] thiophèn-3-yl ce qui correspond aux benzo [b] thiophènes de formule : 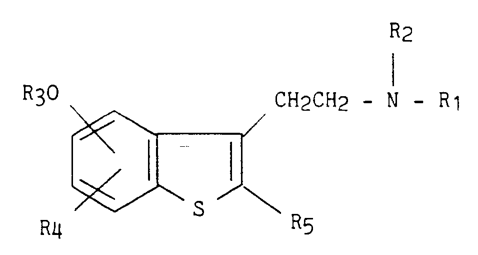 dans laquelle R₁, R₂, R₃, R₄ et R₅ ont la même signification que dans la revendication 1, leurs isomères, épimères, et diastéréoisomères ainsi que leurs sels d'addition à un acide ou à une base pharmaceutiquement acceptable.

4. Composés selon la revendication 1 pour lesquels Ar' représente un noyau benzo [b] furan-3-yl ce qui correspond aux benzo [b] furanes de formule : 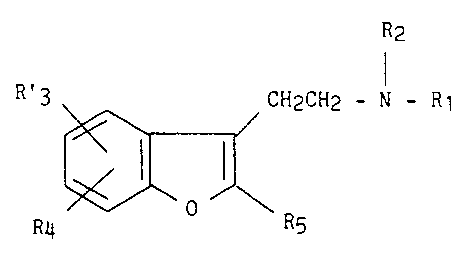 dans laquelle R₁, R₂, R'₃, R₄ et R₅ ont la même signification que dans la revendication 1, leurs isomères, épimères et diastéréoisomères ainsi que leurs sels d'addition à un acide ou à une base pharmaceutiquement acceptable

5. Composés selon la revendication 1 pour lesquels Ar' représente un noyau benzimidazol-1-yl ce qui correspond aux benzimidazoles de formule : 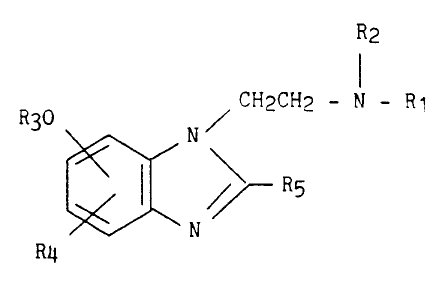 dans laquelle R₁, R₂, R₃, R₄ et R₅ ont la même signification que dans la revendication 1, leurs isomères, épimères, et diastéréoisomères, ainsi que leurs sels d'addition à un acide ou à une base pharmaceutiquement acceptable.

6. Composés selon la revendication 1 pour lesquels Ar' représente un noyau 1,2-benzisothiazol-3-yl ce qui correspond aux 1,2-benzisothiazoles de formule générale : 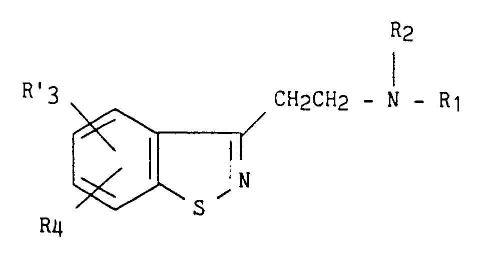 dans laquelle R₁, R₂, R'₃ et R₄ ont la même signification que dans la revendication 1, leurs isomères, épimères, diastéréoisomères, ainsi que leurs sels d'addition à un acide ou à une base pharmaceutiquement acceptable.

7. Composés selon la revendication 1 pour lesquels Ar' représente un noyau 1,2-benzisoxazol-3-yl ce qui correspond aux benzisoxazoles de formule générale : 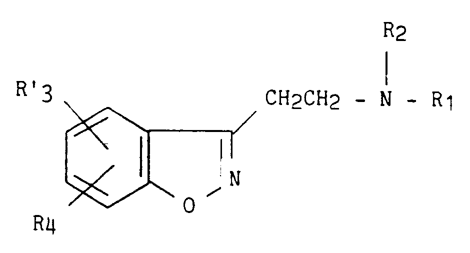 dans laquelle R₁, R₂, R'₃ et R₄ ont la même signification que dans la revendication 1, leurs isomères, épimères, diastéréoisomères, ainsi que leurs sels d'addition à un acide ou à une base pharmaceutiquement acceptable.

8. Composés selon la revendication 1 pour lesquels Ar' représente un noyau indazol-3-yl ce qui correspond aux indazoles de formule générale : 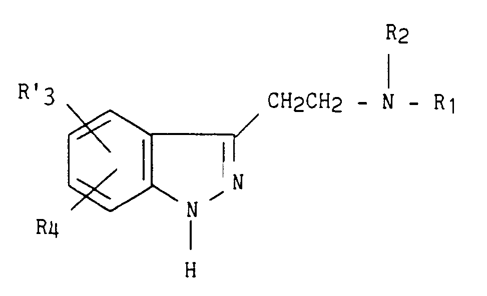 dans laquelle R₁, R₂, R'₃ et R₄ ont la même signification que dans la revendication 1, leurs isomères, épimères, diastéréoisomères, ainsi que leurs sels d'addition à un acide ou une base pharmaceutiquement acceptable.

9. Composé selon la revendication 1 qui est le N-[2-(5-méthoxy indol-3-yl) éthyl] trifluoroacétamide

10. Composé selon la revendication 1 qui est la N-[2-(5-méthoxy indol-3-yl) éthyl[ N'-propylurée.

11. Composé selon la revendication 1 qui est la N-[2-(5-méthoxy indol-3-yl) éthyl] N'-propylthiourée.

12. Composé selon la revendication 1 qui est le N-[2-(5-méthoxy indol-3-yl) éthyl] cyclopropylcarboxamide.

13. Composé selon la revendication 1 qui est la N-[2-(5-méthoxy benzo [b] furan-3-yl) éthyl] N'-propylurée.

14. Composé selon la revendication 1 qui est la N-[2-(5-méthoxy benzo [b] thiophèn-3-yl) éthyl] N'-propylurée.

15. Composé selon la revendication 1 qui est la N-[2-(5-méthoxy indol-3-yl) éthyl] cyclobutylcarboxamide.

16. Composé selon la revendication 1 qui est la N-[2-(6-méthoxy benzimidazol-1-yl) éthyl] acétamide.

17. Composé selon la revendication 1 qui est la N-[2-(6-fluoro 5-méthoxy indol-3-yl) éthyl] cyclopropylcarboxamide.

18. Composé selon la revendication 1 qui est la N-[2-(6-fluoro 5-méthoxy indol-3-yl) éthyl] N'-propylurée.

19. Procédé de préparation des composés de formule **(I)** selon la revendication 1, caractérisé en ce que l'on utilise comme matière première une amine de formule générale **(IX)** :
Ar' - CH₂CH₂ - NHR₂ **(IX)**
dans laquelle Ar' et R₂ ont la même signification que dans la revendication 1, que l'on traite :
- soit par un chlorure d'acide de formule **(X)** : 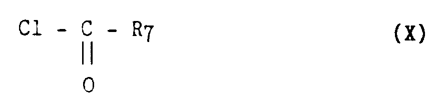 ou par l'anhydride d'acide correspondant de formule **(XI)** : 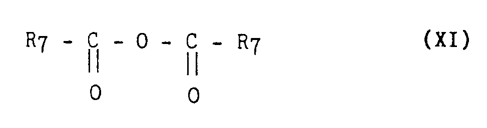 dans lesquelles R₇ a la même signification que dans la revendication 1de manière à obtenir les composés de formule **(Iα)** : 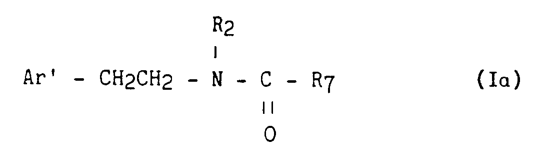 dans laquelle Ar', R₂ et R₇ ont la même signification que dans la revendication 1,
- soit par un isocyanate de formule **(XII)** :
R₈ - N = C = O **(XII)**
dans laquelle R₈ à la même signification que dans la revendication 1, de manière à obtenir les composés de formule **(Iβ)**: 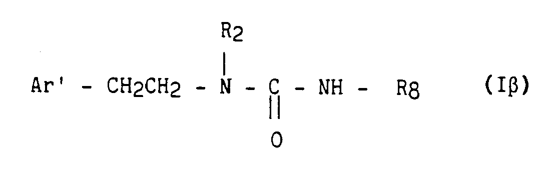 dans laquelle Ar', R₂ et R₈ ont la même signification que dans la revendication 1,
- soit par un isothiocyanate de formule **(XIII)** :
R₈ - N = C = S **(XIII)**
dans laquelle R₈ a la même signification que dans la revendication 1, de manière à obtenir les composés de formule **(Iγ)** : 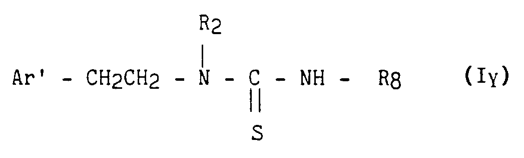 dans laquelle Ar', R₂ et R₈ ont la même signification que dans la revendication 1,
étant entendu que les composés de formules **(Iα)**, **(Iβ)** et **(Iγ)** font partie de l'invention et constituent les composés de formule **(I)** selon la revendication 1,
les composés de formule (I) selon la revendication 1 pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

20. Procédé de préparation des composés de formule (Iε), cas particulier des composés de formule (I) selon la revendication 1 : 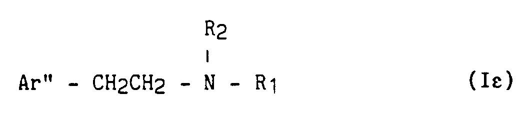 dans laquelle R₁ et R₂ sont tels que définis dans la revendication 1 et Ar'' représente un groupement Ar' tel que défini dans la revendication 1 substitué par un groupement -O-R₃'' avec R₃'' représentant un groupement choisi parmi aryle éventuellement substitué, arylalkyle ou diarylalkyle éventuellement substitués, ou cycloalkyle ou cycloalkylalkyle, (les termes : "aryle", "arylalkyle", "diarylalkyle", "cycloalkyle", "cycloalkylalkyle", et "substitué(s)" étant tels que définis dans la revendication 1),
caractérisé en ce que
on fait réagir un composé de formule (Iε') 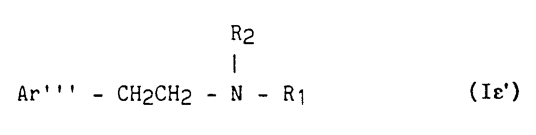 dans laquelle R₁ et R₂ sont tels que définis précédemment et Ar''' représente un groupement Ar' tel que défini dans la revendication 1 substitué par un groupement -O-R₃''' avec R₃'''représentant un atome d'hydrogène, avec un composé de formule (XIV)
R'' - Hal **(XIV)**
dans laquelle Hal représente un atome d'halogène et R'' représente un groupement choisi parmi aryle éventuellement substitué, arylalkyle ou diarylalkyle éventuellement substitués, ou cycloalkyle ou cycloalkylalkyle, (les termes : "aryle", "arylalkyle", "diarylalkyle", "cycloalkyle", "cycloalkylalkyle", et "substitué(s)" étant tels que définis dans la revendication 1),
les composés de formule (Iε) pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

21. Procédé de préparation des composés de formule (Iφ), cas particulier des composés de formule (I) selon la revendication 1 : 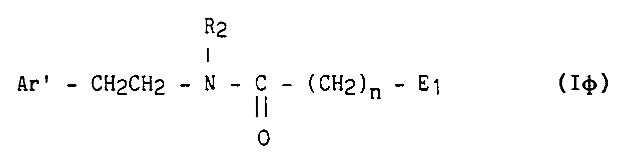 dans laquelle Ar', R₂, E₁, et n sont tels que définis dans la revendication 1, caractérisé en ce que on fait réagir un composé de formule (Iφ') 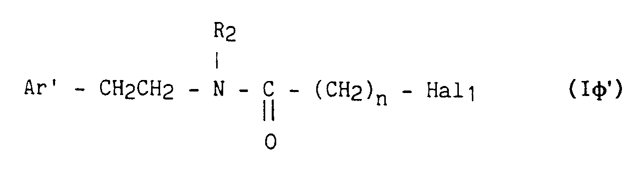 dans laquelle Ar', R₂, et n sont tels que définis dans la revendication 1 et Hal₁ représente un atome d'halogène, avec une morpholine ou une pipérazine non substituée ou substituée par un radical -(CH₂)ₙ'-E₂ où n' et E₂ sont tels que définis dans la revendication 1,
les composés de formule (Iφ) pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

22. Composition pharmaceutique contenant comme principe actif au moins un composé selon l'une des revendications 1 à 18 ou un de ses sels d'addition à un acide ou à une base pharmaceutiquement acceptable, seul ou en combinaison avec un ou plusieurs excipients ou véhicules inertes nontoxiques, pharmaceutiquement acceptables.

23. Composition pharmaceutique selon la revendication 22 utile dans le traitement des troubles du système mélatoninergique.

24. Composition pharmaceutique selon la revendication 22 utile dans le traitement du stress, des troubles du sommeil, de l'anxiété, des dépressions saisonnières, des insomnies et fatigues dues aux décalages horaires, des schizophrénies, de l' attaque de panique, de la mélancolie, de la régulation de l'appétit, de l'insomnie, des troubles psychotiques, de l'épilepsie, de la maladie de Parkinson, de la démence sénile, des désordres liés au vieillissement normal ou pathologique, de la migraine, des pertes de mémoire, de la maladie d'alzheimer, ainsi que des troubles de la circulation cérébrale, de certains cancers, du psoriasis, de l'acné, de la séborrhée ou en tant qu'inhibiteur de l'ovulation ou en médecine vétérinaire dans les troubles du pelage.

## Revendications (Revendications pour l'(les) Etat(s) contractant(s) suivant(s): ES, GR)

1. Procédé de préparation des composés de formule générale **(I)** : 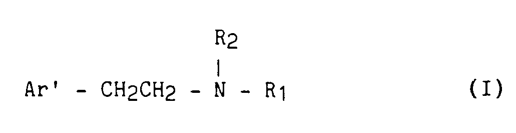 dans laquelle :
- Ar' représente :
- un noyau indol-3-yl de formule **(II)** : 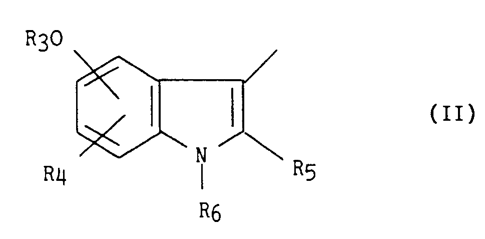
- un noyau benzo [b] thiophèn-3-yl de formule **(III)** : 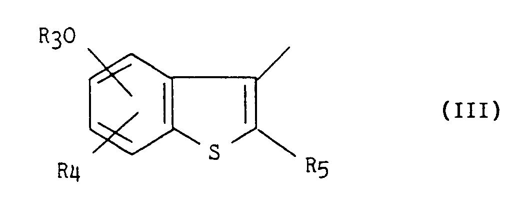
- un noyau benzimidazol-1-yl de formule **(IV)** : 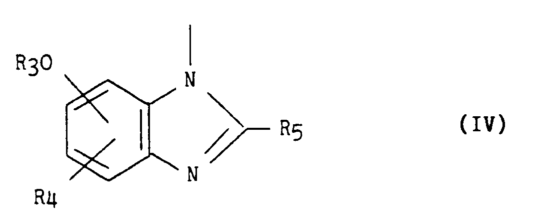
- un noyau benzo [b] furan-3-yl de formule **(V)** : 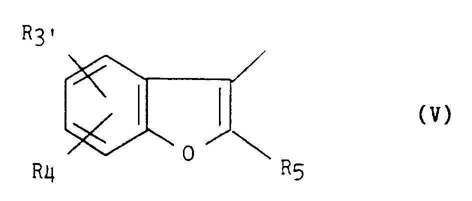
- un noyau 1,2-benzisoxazol-3-yl de formule **(VI)** : 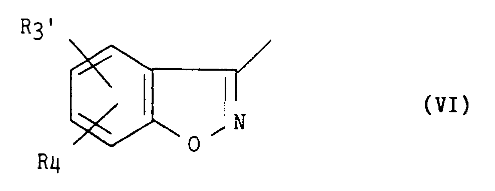
- un noyau 1,2-benzisothiazol-3-yl de formule **(VII)** : 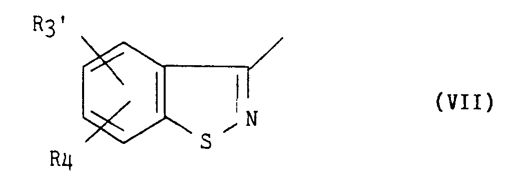
- un noyau indazol-3-yl de formule **(VIII)** : 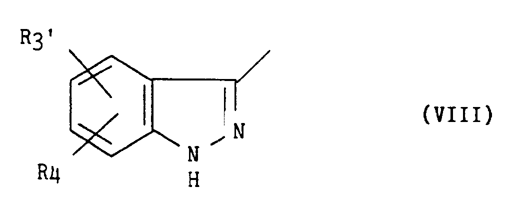
- R₁ représente :
- un groupement 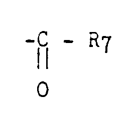 dans lequel R₇ représente un cycloalkyle éventuellement substitué, un cycloalkyl-(C₁-C₄)alkyle éventuellement substitué, ou un trifluorométhyle,
étant entendu que lorsque Ar' représente un groupement choisi parmi ceux de formule (IV), (VI), (VII), et (VIII), alors R₇ peut également représenter un alkyle linéaire ou ramifié de 1 à 6 atomes de carbone non substitué ou substitué par 1 à 2 radicaux halogène,
- un groupement 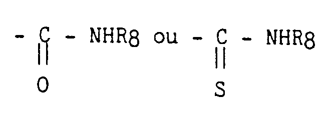 dans lesquels R₈ représente un alkyle inférieur de 1 à 6 atomes de carbone linéaire ou ramifié, un cycloalkyle éventuellement substitué, un cycloalkyl-(C₁-C₄)alkyle éventuellement substitué, un aryle éventuellement substitué, un arylalkyle éventuellement substitué dont la chaine alkyle comporte de 1 à 3 atomes de carbone,
- un groupement 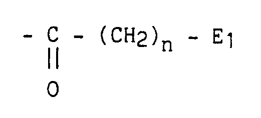 dans lequel
n représente un nombre entier compris entre 1 et 3, et E₁ représente un radical choisi parmi :
- morpholino,
- pipérazine non substituée ou substituée par un radical -(CH₂)ₙ'-E₂ où n' représente un entier de 1 à 4 et E₂ représente un radical phényl ou naphtyl non substitué ou substitué par un à trois radicaux choisis parmi : halogène, (C₁-C₄) alkyl, et (C₁-C₄) alcoxy,
- R₂ représente un hydrogène ou un alkyle inférieur de 1 à 6 atomes de carbone linéaire ou ramifié,
- R₃ représente un hydrogène, un alkyle inférieur de 1 à 6 atomes de carbone linéaire ou ramifié, un aryle éventuellement substitué, un arylalkyle ou diarylalkyle éventuellement substitués dans lesquels la chaîne alkyle comporte de 1 à 3 atomes de carbone, ou un cycloalkyle ou un cycloalkylalkyle dans lequel la chaîne alkyle comporte de 1 à 3 atomes de carbone,
- R₃' représente un atome d'hydrogène ou un groupement -O-R₃ avec R₃ tel que défini précédemment,
- R₄ représente un hydrogène, un halogène, un hydroxy, un alcoxy de 1 à 6 atomes de carbone linéaire ou ramifié, un alkyle inférieur de 1 à 6 atomes de carbone linéaire ou ramifié,
- R₅ représente un hydrogène, un halogène, un alkyle inférieur de 1 à 6 atomes de carbone linéaire ou ramifié, un phényle éventuellement substitué, un phénylalkyle dont la chaîne alkyle comprend de 1 à 3 atomes de carbone, éventuellement substitué,
- R₆ représente un hydrogène, un alkyle inférieur de 1 à 6 atomes de carbone linéaire ou ramifié,
- de leurs isomères, épimères, diastéréoisomères,
- et de leurs sels d'addition à un acide ou à une base pharmaceutiquement acceptable,
- avec les réserves que :
· Ar' ne peut pas représenter un groupement 7-méthoxy benzo [b] furan-3-yl lorsque R₁ représente un cyclopropylcarbonyle,
· R₁ ne peut pas représenter un trifluoroacétyle, lorsque Ar' représente un indole avec R₂ = R₃ = R₄ = R₅ = R₆ = H,
· R₁ ne peut pas représenter un radical anilinothiocarbonyl non substitué ou substitué en position 4 du phényl par un radical alcoxy lorsque Ar' représente un noyau indol-3-yl et R₃ représente un radical méthyle ou benzyle,
· et R₁ ne peut pas représenter un groupement -CO-NH-R₈ lorsque Ar' représente un noyau de formule (II) et R₅ représente un phénylalkyle dont la chaîne alkyle comprend de 1 à 3 atomes de carbone, éventuellement substitué,
étant entendu que, sauf précisions contraires :
- le terme "substitué" associé aux expressions "aryle","arylalkyle", "diarylalkyle", "phényle", et "phénylalkyle" signifie que le ou les noyaux aromatiques peuvent être substitués par un ou plusieurs radicaux choisis parmi : alkyle inférieur de 1 à 6 de carbone linéaire ou ramifié, alcoxy inférieur de 1 à 6 atomes de carbone linéaire ou ramifié, hydroxy, halogène, nitro, et trifluorométhyle,
- le terme "substitué" associé aux expressions "cycloalkyle", et "cycloalkyl-(C₁-C₄)alkyle" signifie que le système cyclique peut être substitué par un ou plusieurs radicaux choisis parmi : halogène, alkyle inférieur de 1 à 6 atomes de carbone, linéaire ou ramifié, et alcoxy inférieur de 1 à 6 atomes de carbone, linéaire ou ramifié,
- le terme "cycloalkyle" désigne un système cyclique, saturé ou insaturé, de 3 à 8 atomes de carbone,
- par groupement aryle on entend groupement pyridyle, phényle, naphtyle, thiényle, furyle, ou pyrimidyle,
caractérisé en ce que :
on utilise comme matière première une amine de formule générale **(IX)** :
Ar' - CH₂CH₂ - NHR₂ **(IX)**
dans laquelle Ar' et R₂ ont la même signification que dans la formule (I), que l'on traite :
- soit par un chlorure d'acide de formule **(X)** : 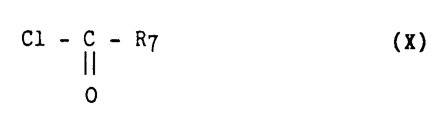 ou par l'anhydride d'acide correspondant de formule **(XI)** : 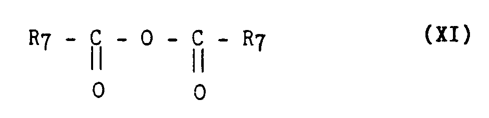 dans lesquelles R₇ a la même signification que dans la formule (I) de manière à obtenir les composés de formule **(Iα)** : 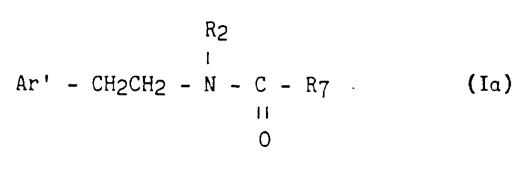 dans laquelle Ar' R₂ et R₇ ont la même signification que dans la formule (I),
- soit par un isocyanate de formule **(XII)** :
R₈ - N = C = O **(XII)**
dans laquelle R₈ à la même signification que dans la formule (I), de manière à obtenir les composés de formule **(Iβ)**: 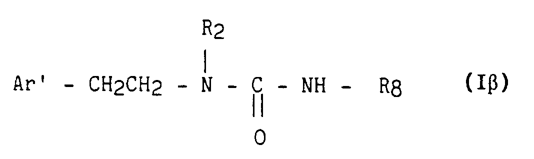 dans laquelle Ar', R₂ et R₈ ont la même signification que dans la formule (I),
- soit par un isothiocyanate de formule **(XIII)** :
R₈ - N = C = S **(XIII)**
dans laquelle R₈ a la même signification que dans la formule (I), de manière à obtenir les composés de formule **(Iγ)** : 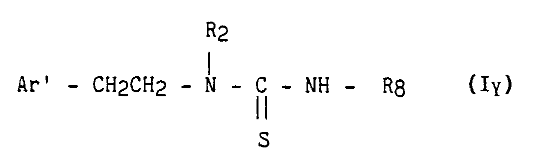 dans laquelle Ar', R₂ et R₈ ont la même signification que dans la formule (I),
étant entendu que les composés de formules **(Iα)**, **(Iβ)** et **(Iγ)** constituent les composés de formule **(I)**,
les composés de formule (I) pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

2. Procédé de préparation des composés de formule (Iε), cas particulier des composés de formule (I) selon la revendication 1 : 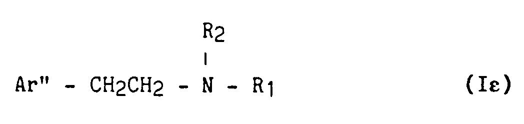 dans laquelle R₁ et R₂ sont tels que définis dans la revendication 1 et Ar'' représente un groupement Ar' tel que défini dans la revendication 1 substitué par un groupement -O-R₃'' avec R₃'' représentant un groupement choisi parmi aryle éventuellement substitué, arylalkyle ou diarylalkyle éventuellement substitués, ou cycloalkyle ou cycloalkylalkyle, (les termes : "aryle", "arylalkyle", "diarylalkyle", "cycloalkyle", "cycloalkylalkyle" et "substitué(s)" étant tels que définis dans la revendication 1),
caractérisé en ce que
on fait réagir un composé de formule (Iε') 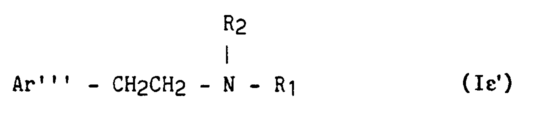 dans laquelle R₁ et R₂ sont tels que définis précédemment et Ar''' représente un groupement Ar' tel que défini dans la revendication 1 substitué par un groupement -O-R₃''' avec R₃'''représentant un atome d'hydrogène, avec un composé de formule (XIV)
R'' - Hal **(XIV)**
dans laquelle Hal représente un atome d'halogène et R'' représente un groupement choisi parmi aryle éventuellement substitué, arylalkyle ou diarylalkyle éventuellement substitués, ou cycloalkyle ou cycloalkylalkyle, (les termes : "aryle", "arylalkyle", "diarylalkyle", "cycloalkyle", "cycloalkylalkyle", et "substitué(s)" étant tels que définis dans la revendication 1),
les composés de formule (Iε) pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

3. Procédé de préparation des composés de formule (Iφ), cas particulier des composés de formule (I) selon la revendication 1 : 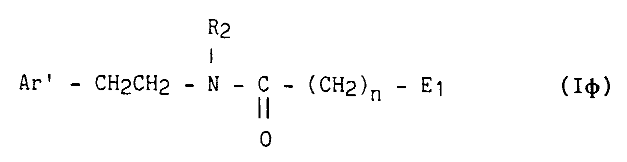 dans laquelle Ar', R₂, E₁, et n sont tels que définis dans la revendication 1, caractérisé en ce que on fait réagir un composé de formule (Iφ') 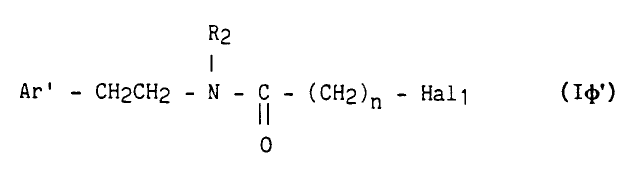 dans laquelle Ar' R₂, et n sont tels que définis dans la revendication 1 et Hal₁ représente un atome d'halogène, avec une morpholine ou une pipérazine non substituée ou substituée par un radical -(CH₂)ₙ'-E₂ où n' et E₂ sont tels que définis dans la revendication 1,
les composés de formule (Iφ) pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

4. Procédé de préparation selon l'une des revendications 1 à 3 des composes pour lesquels Ar' représente un noyau indol-3-yl ce qui correspond aux indoles de formule : 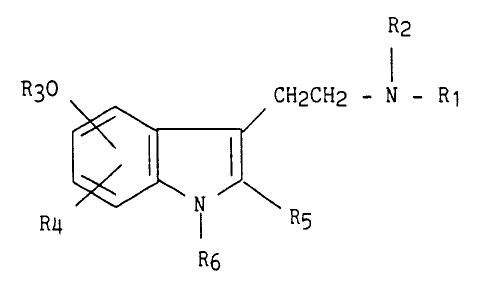 dans laquelle R₁, R₂, R₃, R₄, R₅, et R6 ont la même signification que dans la revendication 1,
de leurs isomères, épimères, et diastéréoisomères, ainsi que de leurs sels d'addition à un acide ou à une base pharmaceutiquement acceptable.

5. Procédé de préparation selon l'une des revendications 1 à 3 des composés pour lesquels Ar' représente un noyau benzo [b] thiophèn-3-yl ce qui correspond aux benzo [b] thiophènes de formule : 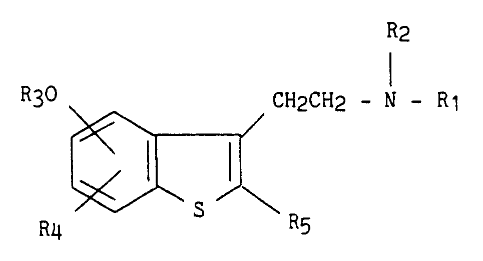 dans laquelle R₁, R₂, R₃, R₄ et R₅ ont la même signification que dans la revendication 1, de leurs isomères, épimères, et diastéréoisomères ainsi que de leurs sels d'addition à un acide ou à une base pharmaceutiquement acceptable.

6. Procédé de préparation selon l'une des revendications 1 à 3 des composés pour lesquels Ar' représente un noyau benzo [b] furan-3-yl ce qui correspond aux benzo [b] furanes de formule : 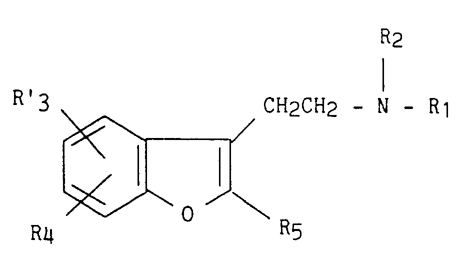 dans laquelle R₁, R₂, R'₃, R₄ et R₅ ont la même signification que dans la revendication 1, de leurs isomères, épimères et diastéréoisomères ainsi que de leurs sels d'addition à un acide ou à une base pharmaceutiquement acceptable.

7. Procédé de préparation selon l'une des revendications 1 à 3 des composés pour lesquels Ar' représente un noyau benzimidazol-1-yl ce qui correspond aux benzimidazoles de formule : 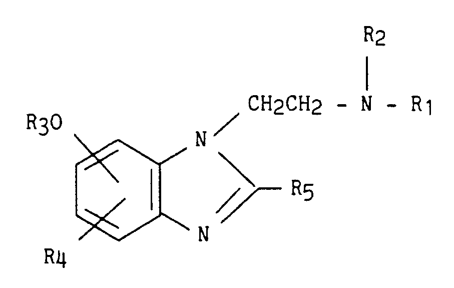 dans laquelle R₁, R₂, R₃, R₄ et R₅ ont la même signification que dans la revendication 1, de leurs isomères, épimères, et diastéréoisomères, ainsi que de leurs sels d'addition à un acide ou à une base pharmaceutiquement acceptable.

8. Procédé de préparation selon l'une des revendications 1 à 3 des composés pour lesquels Ar' représente un noyau 1,2-benzisothiazol-3-yl ce qui correspond aux 1,2-benzisothiazoles de formule générale : 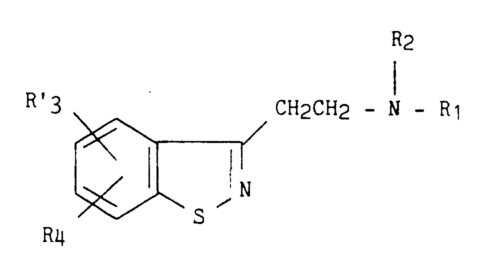 dans laquelle R₁, R₂, R'₃ et R₄ ont la même signification que dans la revendication 1, de leurs isomères, épimères, diastéréoisomères, ainsi que de leurs sels d'addition à un acide ou à une base pharmaceutiquement acceptable.

9. Procédé de préparation selon l'une des revendications 1 à 3 des composés pour lesquels Ar' représente un noyau 1,2-benzisoxazol-3-yl ce qui correspond aux benzisoxazoles de formule générale : 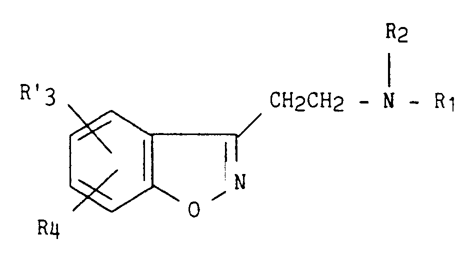 dans laquelle R₁, R₂, R'₃ et R₄ ont la même signification que dans la revendication 1, de leurs isomères, épimères, diastéréoisomères, ainsi que de leurs sels d'addition à un acide ou à une base pharmaceutiquement acceptable.

10. Procédé de préparation selon l'une des revendications 1 à 3 des composés pour lesquels Ar' représente un noyau indazol-3-yl ce qui correspond aux indazoles de formule générale : 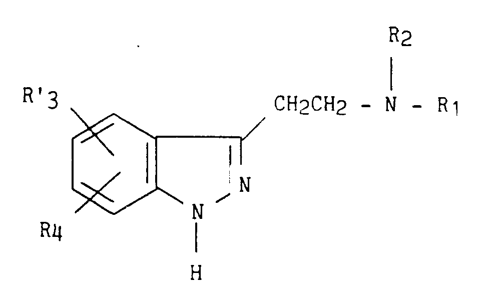 dans laquelle R₁, R₂, R'₃ et R₄ ont la même signification que dans la revendication 1, de leurs isomères, épimères, diastéréoisomères, ainsi que de leurs sels d'addition à un acide ou une base pharmaceutiquement acceptable.

11. Procédé de préparation selon la revendication 1 du composé qui est le N-[2-(5-méthoxy indol-3-yl) éthyl] trifluoroacétamide

12. Procédé de préparation selon la revendication 1 du composé qui est la N-[2-(5-méthoxy indol-3-yl) éthyl] N'-propylurée.

13. Procédé de préparation selon la revendication 1 du composé qui est la N-[2-(5-méthoxy indol-3-yl) éthyl] N'-propylthiourée.

14. Procédé de préparation selon la revendication 1 du composé qui est le N-[2-(5-méthoxy indol-3-yl) éthyl] cyclopropylcarboxamide.

15. Procédé de préparation selon la revendication 1 du composé qui est la N-[2-(5-méthoxy benzo [b] furan-3-yl) éthyl] N'-propylurée.

16. Procédé de préparation selon la revendication 1 du composé qui est la N-[2-(5-méthoxy benzo [b] thiophèn-3-yl) éthyl] N'-propylurée.

17. Procédé de préparation selon la revendication 1 du composé qui est la N-[2-(5-méthoxy indol-3-yl) éthyl] cyclobutylcarboxamide.

18. Procédé de préparation selon la revendication 1 du composé qui est la N-[2-(6-méthoxy benzimidazol-1-yl) éthyl] acétamide.

19. Procédé de préparation selon la revendication 1 du composé qui est la N-[2-(6-fluoro 5-méthoxy indol-3-yl) éthyl] cyclopropylcarboxamide.

20. Procédé de préparation selon la revendication 1 du composé qui est la N-[2-(6-fluoro 5-méthoxy indol-3-yl) éthyl] N'-propylurée.

21. Procédé de préparation d'une composition pharmaceutique contenant comme principe actif au moins un composé préparé selon l'une des revendications 1 à 20 ou un de ses sels d'addition à un acide ou à une base pharmaceutiquement acceptable, seul ou en combinaison avec un ou plusieurs excipients ou véhicules inertes non-toxiques, pharmaceutiquement acceptables.

22. Procédé de préparation d'une composition pharmaceutique selon la revendication 21 utile dans le traitement des troubles du système mélatoninergique.

23. Procédé de préparation d'une composition pharmaceutique selon la revendication 21 utile dans le traitement du stress, des troubles du sommeil, de l'anxiété, des dépressions saisonnières, des insomnies et fatigues dues aux décalages horaires, des schizophrénies, de l' attaque de panique, de la mélancolie, de la régulation de l'appétit, de l'insomnie, des troubles psychotiques, de l'épilepsie, de la maladie de Parkinson, de la démence sénile, des désordres liés au vieillissement normal ou pathologique, de la migraine, des pertes de mémoire, de la maladie d'alzheimer, ainsi que des troubles de la circulation cérébrale, de certains cancers, du psoriasis, de l'acné, de la séborrhée ou en tant qu'inhibiteur de l'ovulation ou en médecine vétérinaire dans les troubles du pelage.

## Claims (Claims for the following Contracting State(s): AT, BE, CH, DE, DK, FR, GB, IE, IT, LI, LU, NL, PT, SE)

1. Compounds of the general formula (I) : 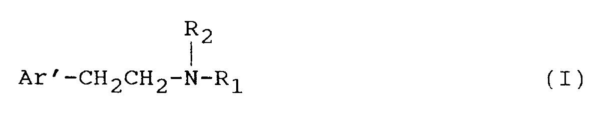 wherein :
- Ar' represents :
- an indol-3-yl ring system of formula (II) : 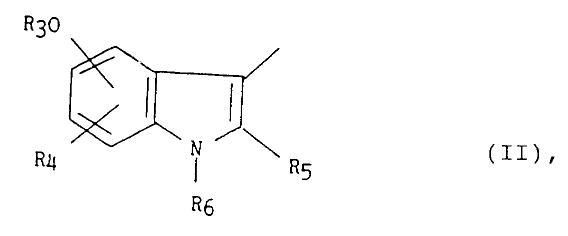
- a benzo[b]thiophen-3-yl ring system of formula (III) : 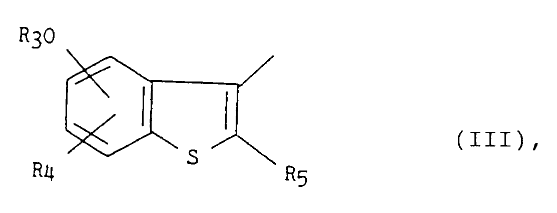
- a benzimidazol-1-yl ring system of formula (IV) : 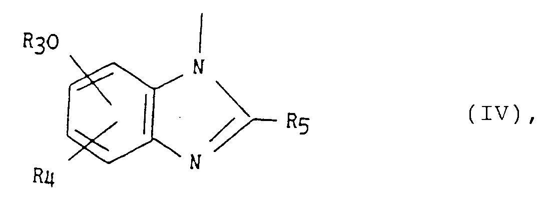
- a benzo[b]furan-3-yl ring system of formula (V) : 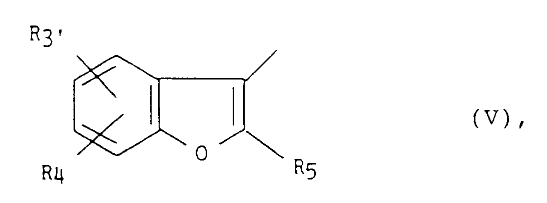
- a 1,2-benzisoxazol-3-yl ring system of formula (VI) : 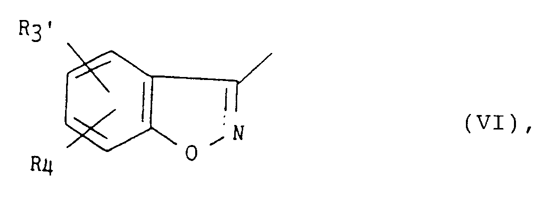
- a 1,2-benzisothiazol-3-yl ring system of formula (VII) : 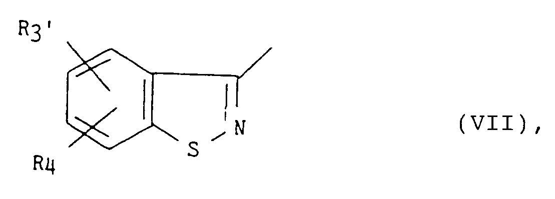
- an indazol-3-yl ring system of formula (VIII) : 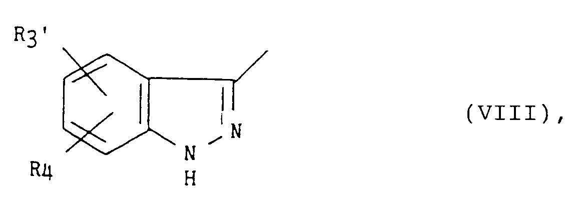
- R₁ represents :
- a 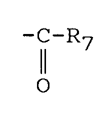 group wherein R₇ represents an optionally substituted cycloalkyl radical, an optionally substituted cycloalkyl-(C₁-C₄)alkyl radical, or a trifluoromethyl radical,
it being understood that, when Ar' represents a group selected from those of formulae (IV), (VI), (VII) and (VIII), R₇ may also represent a straight-chain or branched alkyl radical having from 1 to 6 carbon atoms that is unsubstituted or substituted by 1 or 2 halogen atoms,
- a 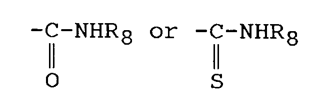 group wherein R₈ represents a a straight-chain or branched lower alkyl radical having from 1 to 6 carbon atoms, an optionally substituted cycloalkyl radical, an optionally substituted cycloalkyl-(C₁-C₄)alkyl radical, an optionally substituted aryl radical, an optionally substituted arylalkyl radical in which the alkyl chain contains from 1 to 3 carbon atoms, or
- a 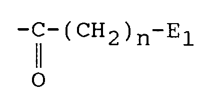 group wherein n represents an integer of from 1 to 3 and E₁ represents a radical selected from:
- morpholino,
- piperazine that is unsubstituted or substituted by a -(CH₂)_{n'}-E₂ radical wherein n' represents an integer of from 1 to 4 and E₂ represents a phenyl or naphthyl radical that is unsubstituted or substituted by from 1 to 3 radicals selected from : halogen, (C₁-C₄)alkyl and (C₁-C₄)alkoxy,
- R₂ represents hydrogen or a straight-chain or branched lower alkyl radical having from 1 to 6 carbon atoms,
- R₃ represents hydrogen, a straight-chain or branched lower alkyl radical having from 1 to 6 carbon atoms, an optionally substituted aryl radical, an optionally substituted arylalkyl or diarylalkyl radical in which the alkyl chain contains from 1 to 3 carbon atoms, or a cycloalkyl or cycloalkylalkyl radical in which the alkyl chain contains from 1 to 3 carbon atoms,
- R₃' represents a hydrogen atom or an -O-R₃ group wherein R₃ is as defined above,
- R₄ represents hydrogen, halogen, a hydroxy radical, a straight-chain or branched alkoxy radical having from 1 to 6 carbon atoms, or a straight-chain or branched lower alkyl radical having from 1 to 6 carbon atoms,
- R₅ represents hydrogen, halogen, a straight-chain or branched lower alkyl radical having from 1 to 6 carbon atoms, an optionally substituted phenyl radical, or an optionally substituted phenylalkyl radical in which the alkyl chain contains from 1 to 3 carbon atoms,
- R₆ represents hydrogen, or a straight-chain or branched lower alkyl radical having from 1 to 6 carbon atoms,
- their isomers, epimers and diastereoisomers,
- and their addition salts with a pharmaceutically acceptable acid or base,
- with the provisos that:
· Ar' cannot represent a 7-methoxybenzo[b]furan-3-yl group when R₁ represents a cyclopropylcarbonyl radical,
· R₁ cannot represent a trifluoroacetyl radical when Ar' represents an indole in which R₂=R₃=R₄=R₅=R₆=H,
· R₁ cannot represent an anilinothiocarbonyl radical that is unsubstituted, or that is substituted in the 4-position of the phenyl ring by an alkoxy radical, when Ar' represents an indol-3-yl ring and R₃ represents a methyl or benzyl radical,
· and R₁ cannot represent a -CO-NH-R₈ group when Ar' represents a ring system of formula (II) and R₅ represents an optionally substituted phenylalkyl radical in which the alkyl chain contains from 1 to 3 carbon atoms,
it being understood that, unless specified otherwise:
- the term "substituted", in connection with the terms "aryl", "arylalkyl", "diarylalkyl", "phenyl" and "phenylalkyl", denote that the aromatic ring or rings may be substituted by one or more radicals selected from: straight-chain or branched lower alkyl having from 1 to 6 carbon atoms, straight-chain or branched lower alkoxy having from 1 to 6 carbon atoms, hydroxy, halogen, nitro and trifluoromethyl,
- the term "substituted" in connection with the terms "cycloalkyl" and "cycloalkyl-(C₁-C₄)alkyl" denotes that the ring may be substituted by one or more radicals selected from : halogen, straight-chain or branched lower alkyl having from 1 to 6 carbon atoms, and straight-chain or branched lower alkoxy having from 1 to 6 carbon atoms,
- the term "cycloalkyl" denotes a saturated or unsaturated ring having from 3 to 8 carbon atoms,
- there is to be understood by "aryl radical" a pyridyl, phenyl, naphthyl, thienyl, furyl or pyrimidyl radical.

2. Compounds according to claim 1, wherein Ar' represents an indol-3-yl ring system, which correspond to indoles of the formula : 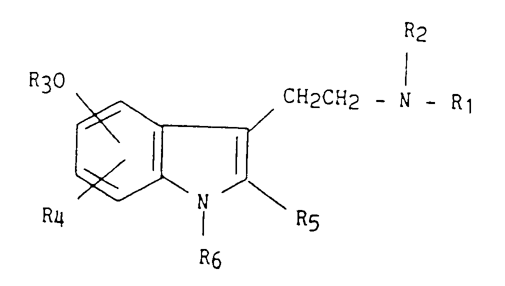 wherein R₁, R₂, R₃, R₄, R₅ and R₆ are as defined in claim 1,
their isomers, epimers and diastereoisomers, and also their addition salts with a pharmaceutically acceptable acid or base.

3. Compounds according to claim 1, wherein Ar' represents a benzo[b]thiophen-3-yl ring system, which correspond to benzo[b]thiophenes of formula : 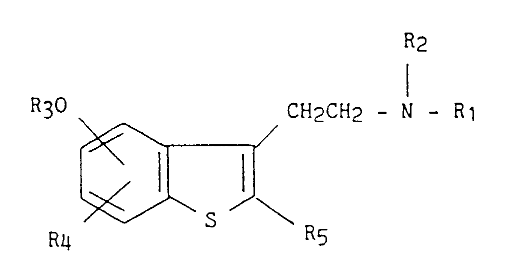 wherein R₁, R₂, R₃, R₄ and R₅ are as defined in claim 1, their isomers, epimers and diastereoisomers, and also their addition salts with a pharmaceutically acceptable acid or base.

4. Compounds according to claim 1, wherein Ar' represents a benzo[b]furan-3-yl ring system, which correspond to benzo[b]furans of formula : 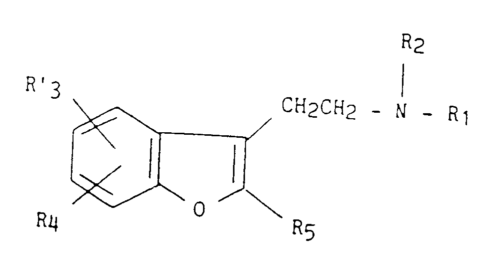 wherein R₁, R₂, R'₃, R₄ and R₅ are as defined in claim 1, their isomers, epimers and diastereoisomers, and also their addition salts with a pharmaceutically acceptable acid or base.

5. Compounds according to claim 1, wherein Ar' represents a benzimidazol-1-yl ring system, which correspond to benzimidazoles of formula : 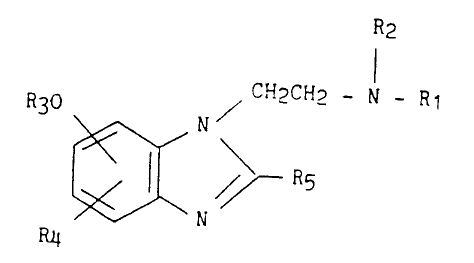 wherein R₁, R₂, R₃, R₄ and R₅ are as defined in claim 1, their isomers, epimers and diastereoisomers, and also their addition salts with a pharmaceutically acceptable acid or base.

6. Compounds according to claim 1, wherein Ar' represents a 1,2-benzisothiazol-3-yl ring system, which correspond to 1,2-benzisothiazoles of the general formula : 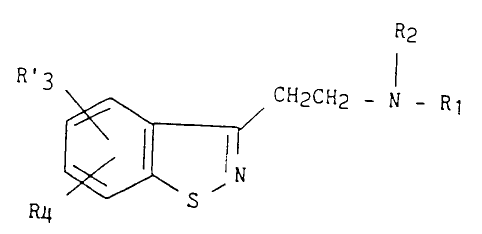 wherein R₁, R₂, R'₃ and R₄ are as defined in claim 1, their isomers, epimers and diastereoisomers, and also their addition salts with a pharmaceutically acceptable acid or base.

7. Compounds according to claim 1, wherein Ar' represents a 1,2-benzisoxazol-3-yl ring system, which correspond to benzisoxazoles of the general formula : 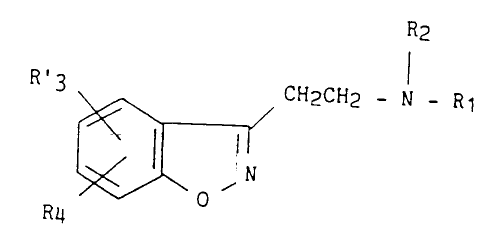 wherein R₁, R₂, R'₃ and R₄ are as defined in claim 1, their isomers, epimers and diastereoisomers, and also their addition salts with a pharmaceutically acceptable acid or base.

8. Compounds according to claim 1, wherein Ar' represents an indazol-3-yl ring system, which correspond to indazoles of the general formula : 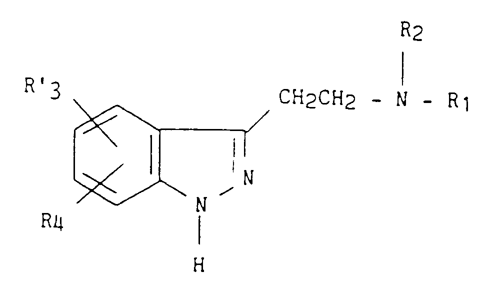 wherein R₁, R₂, R'₃ and R₄ are as defined in claim 1, their isomers, epimers and diastereoisomers, and also their addition salts with a pharmaceutically acceptable acid or base.

9. Compound according to claim 1 which is N-[2-(5-methoxyindol-3-yl)ethyl]trifluoroacetamide.

10. Compound according to claim 1 which is N-[2-(5-methoxyindol-3-yl)ethyl]-N'-propylurea.

11. Compound according to claim 1 which is N-[2-(5-methoxyindol-3-yl)ethyl]-N'-propylthiourea.

12. Compound according to claim 1 which is N-[2-(5-methoxyindol-3-yl)ethyl]cyclopropyl-carboxamide.

13. Compound according to claim 1 which is N-[2-(5-methoxybenzo[b]furan-3-yl)ethyl]-N'-propylurea.

14. Compound according to claim 1 which is N-[2-(5-methoxybenzo[b]thiophen-3-yl)ethyl]-N'-propylurea.

15. Compound according to claim 1 which is N-[2-(5-methoxyindol-3-yl)ethyl]cyclobutyl-carboxamide.

16. Compound according to claim 1 which is N-[2-(6-methoxybenzimidazol-1-yl)ethyl]acetamide.

17. Compound according to claim 1 which is N-[2-(6-fluoro-5-methoxyindol-3-yl)ethyl]cyclopropyl-carboxamide.

18. Compound according to claim 1 which is N-[2-(6-fluoro-5-methoxyindol-3-yl)ethyl]-N'-propylurea.

19. Process for the preparation of compounds of formula (I) according to claim 1, characterised in that there is used as starting material an amine of the general formula (IX) :
Ar'-CH₂CH₂-NHR₂ (IX)
wherein Ar' and R₂ are as defined in claim 1, which is treated :
- either with an acid chloride of formula (X) : 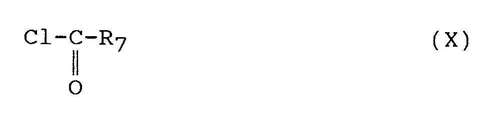 or with the corresponding acid anhydride of formula (XI): 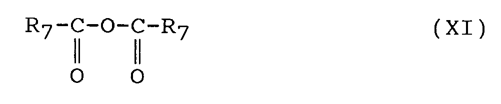 wherein R₇ is as defined in claim 1, to obtain a compound of formula (Iα) 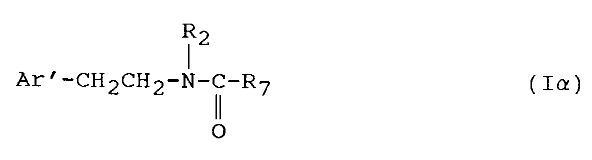 wherein Ar', R₂ and R₇ are as defined in claim 1,
- or with an isocyanate of formula (XII) :
R₈-N=C=O (XII)
wherein R₈ is as defined in claim 1, to obtain a compound of formula (Iβ) 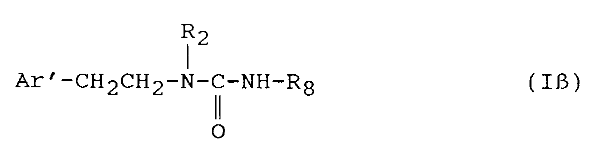 wherein Ar', R₂ and R₈ are as defined in claim 1,
- or with an isothiocyanate of formula (XIII) :
R₈-N=C=S (XIII)
wherein R₈ is as defined in claim 1, to obtain a compound of formula (Iγ): 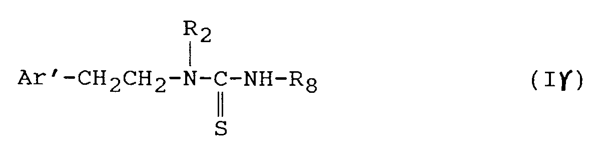 wherein Ar', R₂ and R₈ are as defined in claim 1,
it being understood that the compounds of formula (Iα), (Iβ) and (Iγ) form part of the invention and constitute compounds of formula (I) according to claim 1,
it being possible for the compounds of formula (I) according to claim 1 to be:
- purified according to one or more methods of purification selected from crystallisation, chromatography on a silica column, extraction, filtration and passage over carbon and/or resin,
- separated, where desired, in pure form or in the form of a mixture, into their possible optical isomers,
- and/or converted into salts with a pharmaceutically acceptable acid or base.

20. Process for the preparation of compounds of formula (Iε), a particular instance of compounds of formula (I) according to claim 1 : 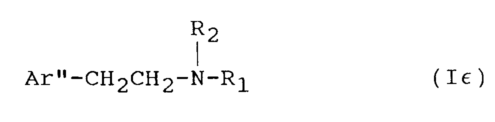 wherein R₁ and R₂ are as defined in claim 1 and Ar'' represents a group Ar' as defined in claim 1 that is substituted by an -O-R₃'' group wherein R₃'' represents a radical selected from optionally substituted aryl, optionally substituted arylalkyl or diarylalkyl, cycloalkyl and cycloalkylalkyl (the terms: "aryl", "arylalkyl", "diarylalkyl", "cycloalkyl", "cycloalkylalkyl" and "substituted" being as defined in claim 1), characterised in that
a compound of formula (Iε') 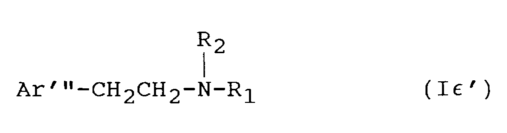 wherein R₁ and R₂ are as defined above and Ar''' represents a group Ar' as defined in claim 1 that is substituted by an -O-R₃''' group wherein R₃''' represents a hydrogen atom, is reacted with a compound of formula (XIV)
R''-Hal (XIV)
wherein Hal represents a halogen atom and R'' represents a radical selected from optionally substituted aryl, optionally substituted arylalkyl or diarylalkyl, cycloalkyl and cycloalkylalkyl (the terms: "aryl", "arylalkyl", "diarylalkyl", "cycloalkyl", "cycloalkylalkyl" and "substituted" being as defined in claim 1),
it being possible for the compounds of formula (Iε) to be:
- purified according to one or more methods of purification selected from crystallisation, chromatography on a silica column, extraction, filtration and passage over carbon and/or resin,
- separated, where desired, in pure form or in the form of a mixture, into their possible optical isomers,
- and/or converted into salts with a pharmaceutically acceptable acid or base.

21. Process for the preparation of compounds of formula (Iφ), a particular instance of compounds of formula (I) according to claim 1 : 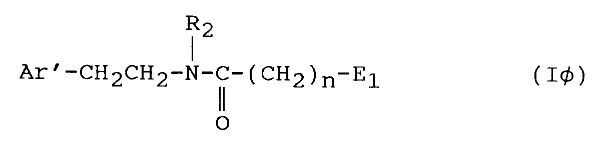 wherein Ar', R₂, E₁ and n are as defined in claim 1, characterised in that a compound of formula (Iφ') 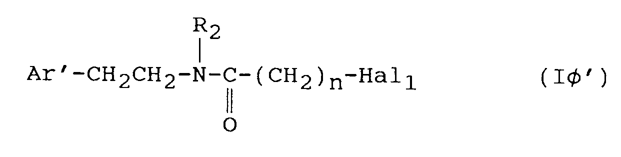 wherein Ar', R₂ and n are as defined in claim 1 and Hal₁ represents a halogen atom, is reacted with a morpholine or a piperazine that is unsubstituted or substituted by a -(CH₂)_{n'}-E₂ radical wherein n' and E₂ are as defined in claim 1,
it being possible for the compounds of formula (Iφ) to be:
- purified according to one or more methods of purification selected from crystallisation, chromatography on a silica column, extraction, filtration and passage over carbon and/or resin,
- separated, where desired, in pure form or in the form of a mixture, into their possible optical isomers,
- and/or converted into salts with a pharmaceutically acceptable acid or base.

22. Pharmaceutical composition comprising as active ingredient at least one compound according to any one of claims 1 to 18 or an addition salt thereof with a pharmaceutically acceptable acid or base, alone or in combination with one or more pharmaceutically acceptable, inert, non-toxic excipients or carriers.

23. Pharmaceutical composition according to claim 22 for use in the treatment of disorders of the melatoninergic system.

24. Pharmaceutical composition according to claim 22 for use in the treatment of stress, sleep disorders, anxiety, seasonal depression, insomnia and fatigue due to shift work, schizophrenia, panic attack, melancholia, appetite regulation, insomnia, psychotic disorders, epilepsy, Parkinson's disease, senile dementia, disorders associated with normal or pathological ageing, migraine, memory loss, Alzheimer's disease, or also cerebral circulation disorders, certain cancers, psoriasis, acne, seborrhoea, or as an ovulation inhibitor or in veterinary medicine for hair-shedding disorders.

## Claims (Claims for the following Contracting State(s): ES, GR)

1. Process for the preparation of compounds of the general formula (I): 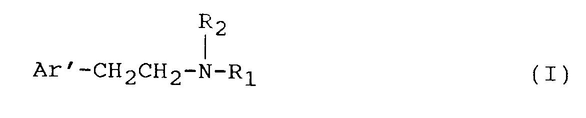 wherein :
- Ar' represents :
- an indol-3-yl ring system of formula (II) : 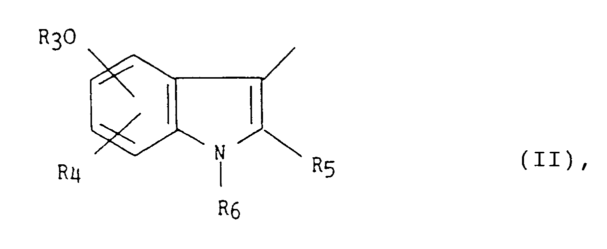
- a benzo[b]thiophen-3-yl ring system of formula (III) : 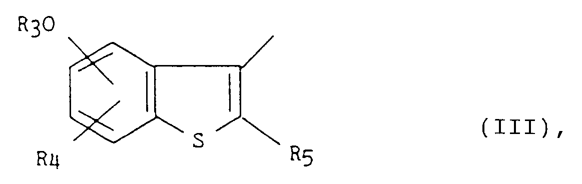
- a benzimidazol-1-yl ring system of formula (IV) : 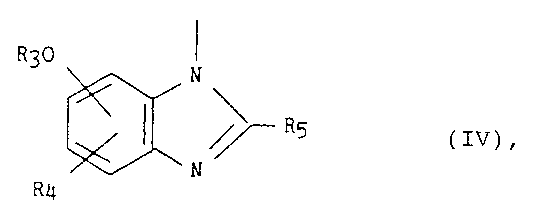
- a benzo[b]furan-3-yl ring system of formula (V) : 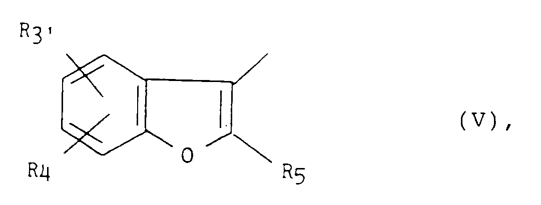
- a 1,2-benzisoxazol-3-yl ring system of formula (VI) : 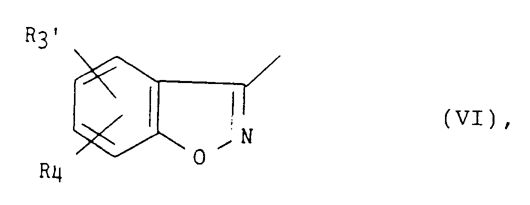
- a 1,2-benzisothiazol-3-yl ring system of formula (VII) : 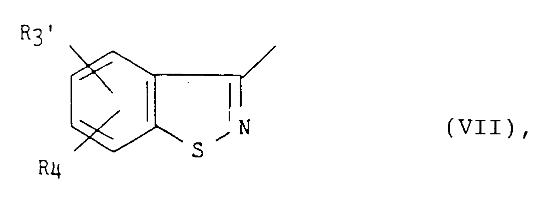
- an indazol-3-yl ring system of formula (VIII) : 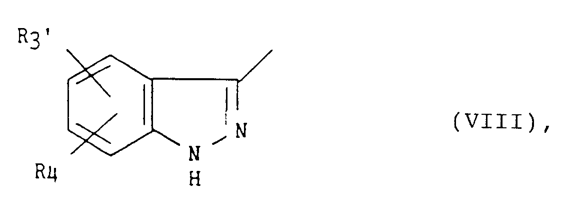
- R₁ represents :
- a 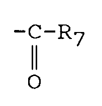 group wherein R₇ represents an optionally substituted cycloalkyl radical, an optionally substituted cycloalkyl-(C₁-C₄)alkyl radical, or a trifluoromethyl radical,
it being understood that, when Ar' represents a group selected from those of formulae (IV), (VI), (VII) and (VIII), R₇ may also represent a straight-chain or branched alkyl radical having from 1 to 6 carbon atoms that is unsubstituted or substituted by 1 or 2 halogen atoms,
- a 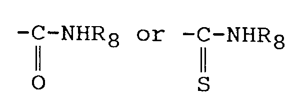 group wherein R₈ represents a a straight-chain or branched lower alkyl radical having from 1 to 6 carbon atoms, an optionally substituted cycloalkyl radical, an optionally substituted cycloalkyl-(C₁-C₄)alkyl radical, an optionally substituted aryl radical, an optionally substituted arylalkyl radical in which the alkyl chain contains from 1 to 3 carbon atoms, or
- a 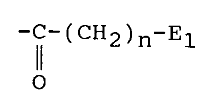 group wherein n represents an integer of from 1 to 3 and E₁ represents a radical selected from:
- morpholino,
- piperazine that is unsubstituted or substituted by a -(CH₂)_{n'}-E₂ radical wherein n' represents an integer of from 1 to 4 and E₂ represents a phenyl or naphthyl radical that is unsubstituted or substituted by from 1 to 3 radicals selected from : halogen, (C₁-C₄)alkyl and (C₁-C₄)alkoxy,
- R₂ represents hydrogen or a straight-chain or branched lower alkyl radical having from 1 to 6 carbon atoms,
- R₃ represents hydrogen, a straight-chain or branched lower alkyl radical having from 1 to 6 carbon atoms, an optionally substituted aryl radical, an optionally substituted arylalkyl or diarylalkyl radical in which the alkyl chain contains from 1 to 3 carbon atoms, or a cycloalkyl or cycloalkylalkyl radical in which the alkyl chain contains from 1 to 3 carbon atoms,
- R₃' represents a hydrogen atom or an -O-R₃ group wherein R₃ is as defined above,
- R₄ represents hydrogen, halogen, a hydroxy radical, a straight-chain or branched alkoxy radical having from 1 to 6 carbon atoms, or a straight-chain or branched lower alkyl radical having from 1 to 6 carbon atoms,
- R₅ represents hydrogen, halogen, a straight-chain or branched lower alkyl radical having from 1 to 6 carbon atoms, an optionally substituted phenyl radical, or an optionally substituted phenylalkyl radical in which the alkyl chain contains from 1 to 3 carbon atoms,
- R₆ represents hydrogen, or a straight-chain or branched lower alkyl radical having from 1 to 6 carbon atoms,
- their isomers, epimers and diastereoisomers,
- and their addition salts with a pharmaceutically acceptable acid or base,
- with the provisos that:
· Ar' cannot represent a 7-methoxybenzo[b]furan-3-yl group when R₁ represents a cyclopropylcarbonyl radical,
· R₁ cannot represent a trifluoroacetyl radical when Ar' represents an indole in which R₂=R₃=R₄=R₅=R₆=H,
· R₁ cannot represent an anilinothiocarbonyl radical that is unsubstituted, or that is substituted in the 4-position of the phenyl ring by an alkoxy radical, when Ar' represents an indol-3-yl ring and R₃ represents a methyl or benzyl radical,
· and R₁ cannot represent a -CO-NH-R₈ group when Ar' represents a ring system of formula (II) and R₅ represents an optionally substituted phenylalkyl radical in which the alkyl chain contains from 1 to 3 carbon atoms,
it being understood that, unless specified otherwise:
- the term "substituted", in connection with the terms "aryl", "arylalkyl", "diarylalkyl", "phenyl" and "phenylalkyl", denote that the aromatic ring or rings may be substituted by one or more radicals selected from: straight-chain or branched lower alkyl having from 1 to 6 carbon atoms, straight-chain or branched lower alkoxy having from 1 to 6 carbon atoms, hydroxy, halogen, nitro and trifluoromethyl,
- the term "substituted" in connection with the terms "cycloalkyl" and "cycloalkyl-(C₁-C₄)alkyl" denotes that the ring may be substituted by one or more radicals selected from : halogen, straight-chain or branched lower alkyl having from 1 to 6 carbon atoms, and straight-chain or branched lower alkoxy having from 1 to 6 carbon atoms,
- the term "cycloalkyl" denotes a saturated or unsaturated ring having from 3 to 8 carbon atoms,
- there is to be understood by "aryl radical" a pyridyl, phenyl, naphthyl, thienyl, furyl or pyrimidyl radical,
characterised in that there is used as starting material an amine of the general formula (IX) :
Ar'-CH₂CH₂-NHR₂ (IX)
wherein Ar' and R₂ are as defined in claim 1, which is treated :
- either with an acid chloride of formula (X) : 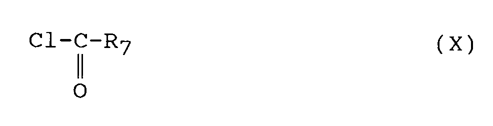 or with the corresponding acid anhydride of formula (XI): 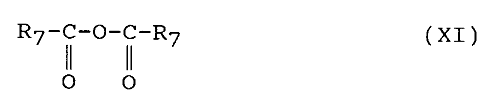 wherein R₇ is as defined in claim 1, to obtain a compound of formula (Iα) 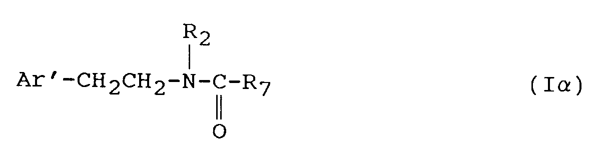 wherein Ar', R₂ and R₇ are as defined for formula (I),
- or with an isocyanate of formula (XII) :
R₈-N=C=O (XII)
wherein R₈ is as defined for formula (I), to obtain a compound of formula (Iβ) 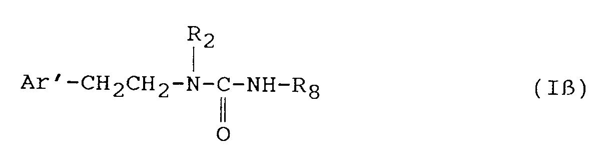 wherein Ar', R₂ and R₈ are as defined for formula (I),
- or with an isothiocyanate of formula (XIII) :
R₈-N=C=S (XIII)
wherein R₈ is as defined for formula (I), to obtain a compound of formula (Iγ): 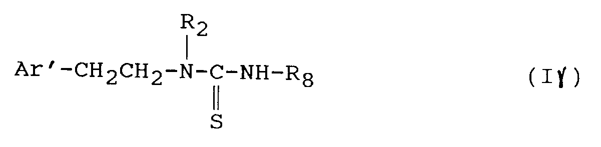 wherein Ar', R₂ and R₈ are as defined for formula (I),
it being understood that the compounds of formula (Iα), (Iβ) and (Iγ) form part of the invention and constitute compounds of formula (I),
it being possible for the compounds of formula (I) to be:
- purified according to one or more methods of purification selected from crystallisation, chromatography on a silica column, extraction, filtration and passage over carbon and/or resin,
- separated, where desired, in pure form or in the form of a mixture, into their possible optical isomers,
- and/or converted into salts with a pharmaceutically acceptable acid or base.

2. Process for the preparation of compounds of formula (Iε), a particular instance of compounds of formula (I) according to claim 1 : 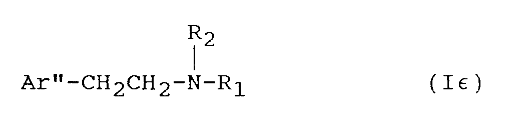 wherein R₁ and R₂ are as defined in claim 1 and Ar'' represents a group Ar' as defined in claim 1 that is substituted by an -O-R₃'' group wherein R₃'' represents a radical selected from optionally substituted aryl, optionally substituted arylalkyl or diarylalkyl, cycloalkyl and cycloalkylalkyl (the terms: "aryl", "arylalkyl", "diarylalkyl", "cycloalkyl", "cycloalkylalkyl" and "substituted" being as defined in claim 1),
characterised in that
a compound of formula (Iε') 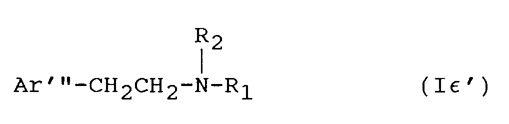 wherein R₁ and R₂ are as defined above and Ar''' represents a group Ar' as defined in claim 1 that is substituted by an -O-R₃''' group wherein R₃''' represents a hydrogen atom, is reacted with a compound of formula (XIV)
R''-Hal (XIV)
wherein Hal represents a halogen atom and R'' represents a radical selected from optionally substituted aryl, optionally substituted arylalkyl or diarylalkyl, cycloalkyl and cycloalkylalkyl (the terms: "aryl", "arylalkyl", "diarylalkyl", "cycloalkyl", "cycloalkylalkyl" and "substituted" being as defined in claim 1),
it being possible for the compounds of formula (Iε) to be:
- purified according to one or more methods of purification selected from crystallisation, chromatography on a silica column, extraction, filtration and passage over carbon and/or resin,
- separated, where desired, in pure form or in the form of a mixture, into their possible optical isomers,
- and/or converted into salts with a pharmaceutically acceptable acid or base.

3. Process for the preparation of compounds of formula (Iφ), a particular instance of compounds of formula (I) according to claim 1 : 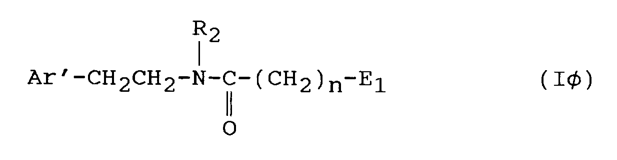 wherein Ar', R₂, E₁ and n are as defined in claim 1, characterised in that a compound of formula (Iφ') 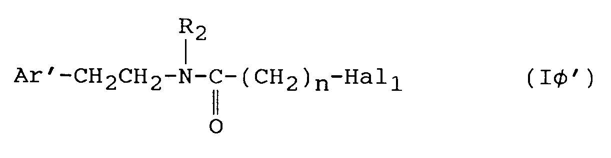 wherein Ar', R₂ and n are as defined in claim 1 and Hal₁ represents a halogen atom, is reacted with a morpholine or a piperazine that is unsubstituted or substituted by a -(CH₂)_{n'}-E₂ radical wherein n' and E₂ are as defined in claim 1,
it being possible for the compounds of formula (Iφ) to be:
- purified according to one or more methods of purification selected from crystallisation, chromatography on a silica column, extraction, filtration and passage over carbon and/or resin,
- separated, where desired, in pure form or in the form of a mixture, into their possible optical isomers,
- and/or converted into salts with a pharmaceutically acceptable acid or base.

4. Process according to any one of claims 1 to 3 for the preparation of compounds wherein Ar' represents an indol-3-yl ring system, which correspond to indoles of formula: 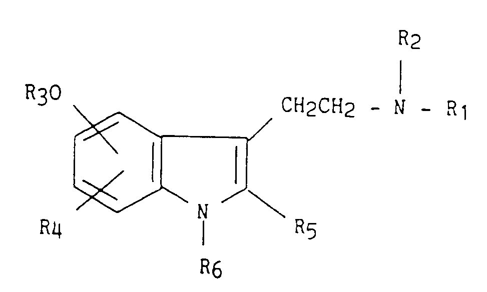 wherein R₁, R₂, R₃, R₄, R₅ and R₆ are as defined in claim 1,
their isomers, epimers and diastereoisomers, and also their addition salts with a pharmaceutically acceptable acid or base.

5. A process according to any one of claims 1 to 3 for the preparation of compounds wherein Ar' represents a benzo[b]thiophen-3-yl ring system, which correspond to benzo[b]thiophenes of formula : 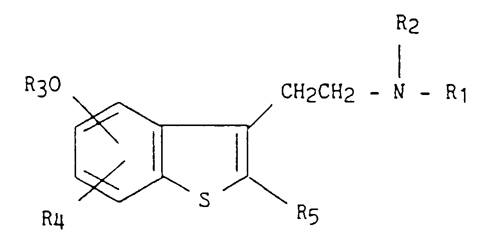 wherein R₁, R₂, R₃, R₄ and R₅ are as defined in claim 1, their isomers, epimers and diastereoisomers, and also their addition salts with a pharmaceutically acceptable acid or base.

6. Process according to any one of claims 1 to 3 for the preparation of compounds wherein Ar' represents a benzo[b]furan-3-yl ring system, which correspond to benzo[b]furans of formula : 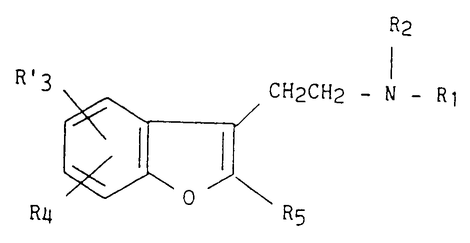 wherein R₁, R₂, R'₃, R₄ and R₅ are as defined in claim 1, their isomers, epimers and diastereoisomers, and also their addition salts with a pharmaceutically acceptable acid or base.

7. Process according to any one of claims 1 to 3 for the preparation of compounds wherein Ar' represents a benzimidazol-1-yl ring system, which correspond to benzimidazoles of formula : 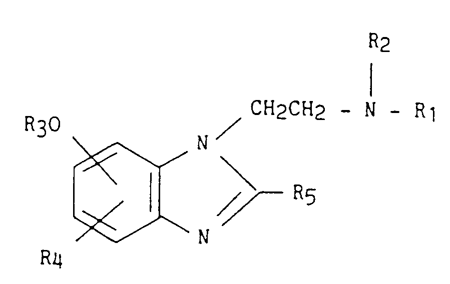 wherein R₁, R₂, R₃, R₄ and R₅ are as defined in claim 1, their isomers, epimers and diastereoisomers, and also their addition salts with a pharmaceutically acceptable acid or base.

8. Process according to any one of claims 1 to 3 for the preparation of compounds wherein Ar' represents a 1,2-benzisothiazol-3-yl ring system, which correspond to 1,2-benzisothiazoles of the general formula : 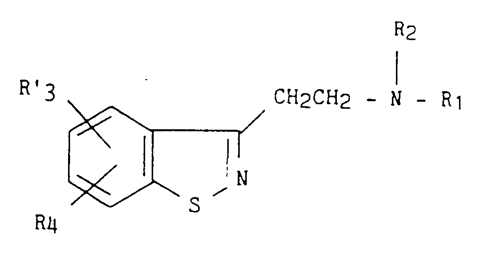 wherein R₁, R₂, R'₃ and R₄ are as defined in claim 1, their isomers, epimers and diastereoisomers, and also their addition salts with a pharmaceutically acceptable acid or base.

9. Process according to any one of claims 1 to 3 for the preparation of compounds wherein Ar' represents a 1,2-benzisoxazol-3-yl ring system, which correspond to benzisoxazoles of the general formula : 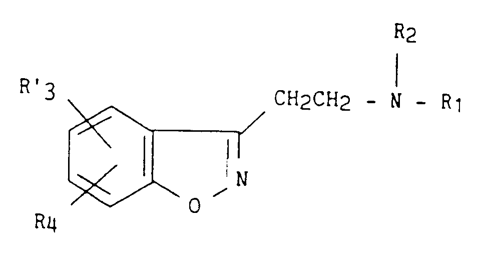 wherein R₁, R₂, R'₃ and R₄ are as defined in claim 1, their isomers, epimers and diastereoisomers, and also their addition salts with a pharmaceutically acceptable acid or base.

10. Process according to any one of claims 1 to 3 for the preparation of compounds wherein Ar' represents an indazol-3-yl ring system, which correspond to indazoles of the general formula : 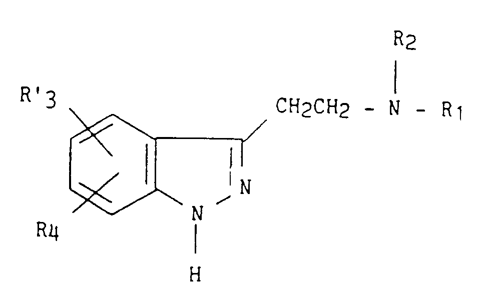 wherein R₁, R₂, R'₃ and R₄ are as defined in claim 1, their isomers, epimers and diastereoisomers, and also their addition salts with a pharmaceutically acceptable acid or base.

11. Process according to claim 1 for the preparation of the compound N-[2-(5-methoxyindol-3-yl)ethyl]trifluoroacetamide.

12. Process according to claim 1 for the preparation of the compound N-[2-(5-methoxyindol-3-yl)ethyl]-N'-propylurea.

13. Process according to claim 1 for the preparation of the compound N-[2-(5-methoxyindol-3-yl)ethyl]-N'-propylthiourea.

14. Process according to claim 1 for the preparation of the compound N-[2-(5-methoxyindol-3-yl)ethyl]cyclopropyl-carboxamide.

15. Process according to claim 1 for the preparation of the compound N-[2-(5-methoxybenzo[b]furan-3-yl)ethyl]-N'-propylurea.

16. Process according to claim 1 for the preparation of the compound N-[2-(5-methoxybenzo[b]thiophen-3-yl)ethyl]-N'-propylurea.

17. Process according to claim 1 for the preparation of the compound N-[2-(5-methoxyindol-3-yl)ethyl]cyclobutyl-carboxamide.

18. Process according to claim 1 for the preparation of the compound N-[2-(6-methoxybenzimidazol-1-yl)ethyl]-acetamide.

19. Process according to claim 1 for the preparation of the compound N-[2-(6-fluoro-5-methoxyindol-3-yl)-ethyl]cyclopropyl-carboxamide.

20. Process according to claim 1 for the preparation of the compound N-[2-(6-fluoro-5-methoxyindol-3-yl)-ethyl]-N'-propylurea.

21. Process for the preparation of a pharmaceutical composition comprising as active ingredient at least one compound according to any one of claims 1 to 20 or an addition salt thereof with a pharmaceutically acceptable acid or base, alone or in combination with one or more pharmaceutically acceptable, inert, non-toxic excipients or carriers.

22. Process according to claim 21 for the preparation of a pharmaceutical composition for use in the treatment of disorders of the melatoninergic system.

23. Process according to claim 21 for the preparation of a pharmaceutical composition for use in the treatment of stress, sleep disorders, anxiety, seasonal depression, insomnia and fatigue due to shift work, schizophrenia, panic attack, melancholia, appetite regulation, insomnia, psychotic disorders, epilepsy, Parkinson's disease, senile dementia, disorders associated with normal or pathological ageing, migraine, memory loss, Alzheimer's disease, or also cerebral circulation disorders, certain cancers, psoriasis, acne, seborrhoea, or as an ovulation inhibitor or in veterinary medicine for hair-shedding disorders.

## Patentansprüche (Patentansprüche für folgende(n) Vertragsstaat(en): AT, BE, CH, DE, DK, FR, GB, IE, IT, LI, LU, NL, PT, SE)

1. Verbindungen der allgemeinen Formel (I): 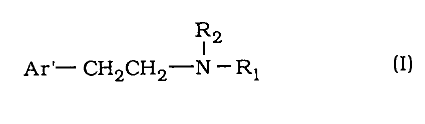 in der:
- Ar':
- einen Indol-3-yl-kern der Formel (II): 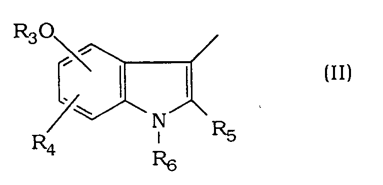
- einen Benzo[b]thiophen-3-yl-kern der Formel (III): 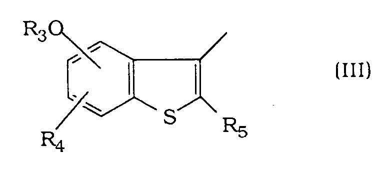
- einen Benzimidazol-1-yl-kern der Formel (IV): 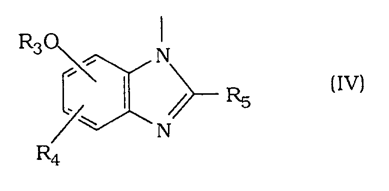
- einen Benzo[b]furan-3-yl-kern der Formel (V): 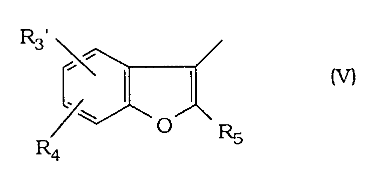
- einen 1,2-Benzisoxazol-3-yl-kern der Formel (VI): 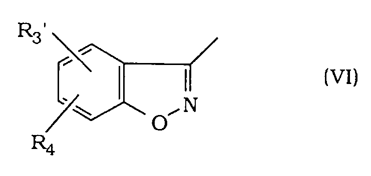
- einen 1,2-Benzisothiazol-3-yl-kern der Formel (VII): 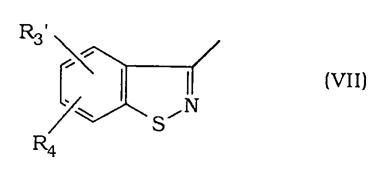 oder
- einen Indazol-3-yl-kern der Formel (VIII): 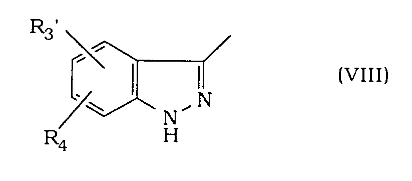
- R₁:
- eine Gruppe 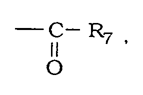 in der R₇ eine gegebenenfalls substituierte Cycloalkylgruppe, eine gegebenenfalls substituierte Cycloalkyl-(C₁-C₄)-alkylgruppe oder eine Trifluormethylgruppe darstellt,
mit der Maßgabe, daß, wenn Ar' eine Gruppe ausgewählt aus den Gruppen der Formeln (IV), (VI), (VII) und (VIII) darstellt, R₇ auch eine geradkettige oder verzweigte Alkylgruppe mit 1 bis 6 Kohlenstoffatomen, die nichtsubstituiert oder mit 1 bis 2 Halogenresten substituiert sein kann, darstellen kann,
- eine Gruppe 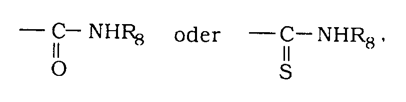 in der R₈ eine geradkettige oder verzweigte Niedrigalkylgruppe mit 1 bis 6 Kohlenstoffatomen, eine gegebenenfalls substituierte Cycloalkylgruppe, eine gegebenenfalls substituierte Cycloalkyl-(C₁-C₄)-alkylgruppe, eine gegebenenfalls substituierte Arylgruppe oder eine gegebenenfalls substituierte Arylalkylgruppe, deren Alkylkette 1 bis 3 Kohlenstoffatome aufweist, darstellt,
- eine Gruppe 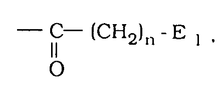 in der
n eine ganze Zahl mit einem Wert zwischen 1 und 3 und E₁ einen Rest ausgewählt aus:
- Morpholino,
- Piperazin, der nichtsubstituiert oder durch eine Gruppe-(CH₂)_{n'}-E₂ substituiert ist, worin n' eine ganze Zahl mit einem Wert von 1 bis 4 und E₂ eine Phenyl- oder Naphthylgruppe, die nichtsubstituiert oder durch einen bis drei Reste ausgewählt aus Halogenatomen, (C₁-C₄)-Alkylgruppen und (C₁-C₄)-Alkoxygruppen substituiert ist, bedeuten,
darstellen,
- R₂ ein Wasserstoffatom oder eine geradkettige oder verzweigte Niedrigalkylgruppe mit 1 bis 6 Kohlenstoffatomen.
- R₃ ein Wasserstoffatom, eine geradkettige oder verzweigte Niedrigalkylgruppe mit 1 bis 6 Kohlenstoffatomen, eine gegebenenfalls substituierte Arylgruppe, eine gegebenenfalls substituierte Arylalkyl- oder Diarylalkylgruppe, worin die Alkylkette 1 bis 3 Kohlenstoffatome aufweist, oder eine Cycloalkylgruppe oder eine Cycloalkylalkylgruppe, worin die Alkylkette 1 bis 3 Kohlenstoffatome aufweist,
- R₃' ein Wasserstoffatom oder eine Gruppe -O-R₃, worin R₃ die oben angegebenen Bedeutungen besitzt,
- R₄ ein Wasserstoffatom, ein Halogenatom, eine Hydroxylgruppe, eine geradkettige oder verzweigte Alkoxygruppe mit 1 bis 6 Kohlenstoffatomen oder eine geradkettige oder verzweigte Niedrigalkylgruppe mit 1 bis 6 Kohlenstoffatomen,
- R₅ ein Wasserstoffatom, ein Halogenatom, eine geradkettige oder verzweigte Niedrigalkylgruppe mit 1 bis 6 Kohlenstoffatomen, eine gegebenenfalls substituierte Phenylgruppe oder eine gegebenenfalls substituierte Phenylalkylgruppe, deren Alkylkette 1 bis 3 Kohlenstoffatome aufweist,
- R₆ ein Wasserstoffatom oder eine geradkettige oder verzweigte Niedrigalkylgruppe mit 1 bis 6 Kohlenstoffatomen,
bedeuten,
- deren Isomere, Epimere und Diastereoisomere,
- und deren Additionssalze mit einer pharmazeutisch annehmbaren Säure oder Base,
- mit der Maßgabe daß:
· Ar' keine 7-Methoxy-benzo[b]furan-3-yl-gruppe darstellt, wenn R₁ eine Cyclopropylcarbonylgruppe bedeutet,
· R₁ keine Trifluoracetylgruppe darstellt, wenn Ar' einen Indolrest darstellt, worin R₂ = R₃ = R₄ = R₅ = R₆ = H bedeuten,
· R₁ keine Anilinothiocarbonylgruppe darstellt, die nichtsubstituiert oder in der 4-Stellung des Phenylrests durch eine Alkoxygruppe substituiert ist, wenn Ar' einen Indol-3-yl-kern und R₃ eine Methyl- oder Benzylgruppe bedeuten,
· und R₁ keine Gruppe -CO-NH-R₈ darstellt, wenn Ar' einen Kern der Formel (II) und R₅ eine gegebenenfalls substituierte Phenylalkylgruppe, deren Alkylkette 1 bis 3 Kohlenstoffatome aufweist, bedeuten,
wobei, wenn nichts anderes angegeben ist:
- der mit den Begriffen "Arylgruppe", "Arylalkylgruppe". "Diarylalkylgruppe", "Phenylgruppe" und "Phenylalkylgruppe" verknüpfte Ausdruck "substituiert" bedeutet, daß der oder die aromatischen Kerne durch eine oder mehrere Gruppen ausgewählt aus: geradkettigen oder verzweigten Niedrigalkylgruppen mit 1 bis 6 Kohlenstoffatomen, geradkettigen oder verzweigten Niedrigalkoxygruppen mit 1 bis 6 Kohlenstoffatomen, Hydroxylgruppen, Halogenatomen, Nitrogruppen und Trifluormethylgruppen substituiert sein können,
- der mit den Bezeichnungen "Cycloalkylgruppe" und "Cycloalkyl-(C₁-C₄)-alkylgruppe" verknüpfte Ausdruck "substituiert" bedeutet, daß das cyclische System durch einen oder mehrere Reste ausgewählt aus Halogenatomen, geradkettigen oder verzweigten Niedrigalkylgruppen mit 1 bis 6 Kohlenstoffatomen und geradkettigen oder verzweigten Niedrigalkoxygruppen mit 1 bis 6 Kohlenstoffatomen substituiert sein können,
- der Begriff "Cycloalkylgruppe" für ein gesättigtes oder ungesättigtes cyclisches System mit 3 bis 8 Kohlenstoffatomen steht und
- unter einer "Arylgruppe" eine Pyridyl-, Phenyl-, Naphthyl-, Thienyl-, Furyl- oder Pyrimidyl-gruppe zu verstehen ist.

2. Verbindungen nach Anspruch 1, worin Ar' einen Indol-3-yl-kern bedeutet, der Indolen der Formel entspricht: 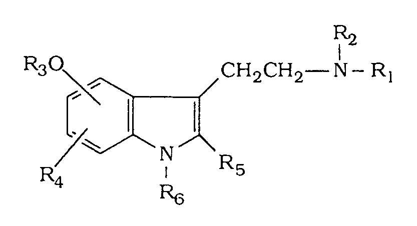 in der R₁, R₂, R₃, R₄, R₅ und R₆ die in Anspruch 1 angegebenen Bedeutungen besitzen,
deren Isomere, Epimere und Diastereoisomere sowie deren Additionssalze mit einer pharmazeutisch annehmbaren Säure oder Base.

3. Verbindung nach Anspruch 1, worin Ar' einen Benzo[b]thiophen-3-yl-kern darstellt, der Benzo[b]thiophenen der Formel entspricht: 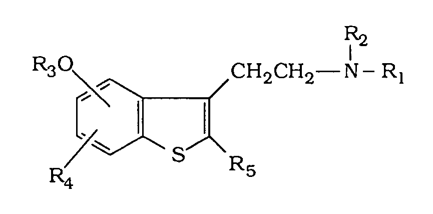 in der R₁, R₂, R₃, R₄ und R₅ die in Anspruch 1 angegebenen Bedeutungen besitzen, deren Isomere, Epimere und Diastereoisomere sowie deren Additionssalze mit einer pharmazeutisch annehmbaren Säure oder Base.

4. Verbindungen nach Anspruch 1, worin Ar' einen Benzo[b]furan-3-yl-kern bedeutet, der Benzo[b]furanen der Formel entspricht: 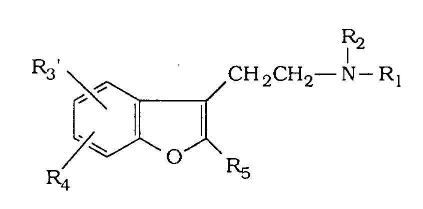 in der R₁, R₂, R₃', R₄ und R₅ die in Anspruch 1 angegebenen Bedeutungen besitzen, deren Isomere, Epimere und Diastereoisomere sowie deren Additionssalze mit einer pharmazeutisch annehmbaren Säure oder Base.

5. Verbindungen nach Anspruch 1, worin Ar' einen Benzimidazol-1-yl-kern bedeutet, der Benzimidazolen der Formel entspricht: 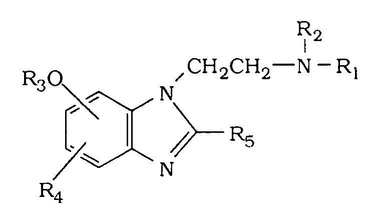 in der R₁, R₂, R₃, R₄ und R₅ die in Anspruch 1 angegebenen Bedeutungen besitzen, deren Isomere, Epimere und Diastereoisomere sowie deren Additionssalze mit einer pharmazeutisch annehmbaren Säure oder Base.

6. Verbindungen nach Anspruch 1, worin Ar' einen 1,2-Benzisothiazol-3-yl-kern darstellt, der 1,2-Benzisothiazolen der allgemeinen Formel entspricht: 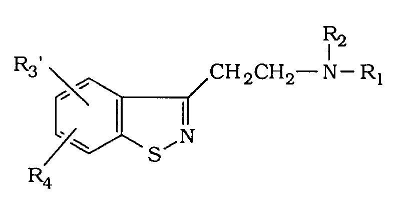 in der R₁, R₂, R₃' und R₄ die in Anspruch 1 angegebenen Bedeutungen besitzen, deren Isomere, Epimere und Diastereoisomere sowie deren Additionssalze mit einer pharmazeutisch annehmbaren Säure oder Base.

7. Verbindungen nach Anspruch 1, worin Ar' einen 1,2-Benzisoxazol-3-yl-kern darstellt, der Benzisoxazolen der allgemeinen Formel entspricht: 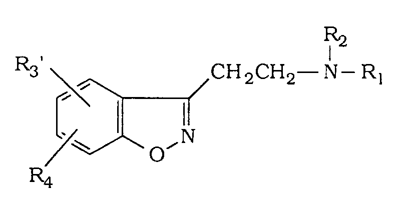 in der R₁, R₂, R₃' und R₄ die in Anspruch 1 angegebenen Bedeutungen besitzen, deren Isomere, Epimere und Diastereoisomere sowie deren Additionssalze mit einer pharmazeutisch annehmbaren Säure oder Base.

8. Verbindungen nach Anspruch 1, worin Ar' einen Indazol-3-yl-kern darstellt, der Indazolen der allgemeinen Formel entspricht: 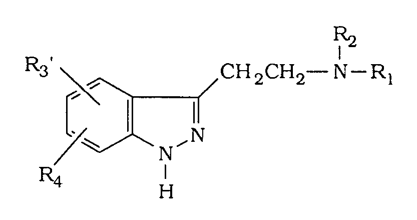 In der R₁, R₂, R₃' und R₄ die in Anspruch 1 angegebenen Bedeutungen besitzen, deren Isomere. Epimere und Diastereoisomere sowie deren Additionssalze mit einer pharmazeutisch annehmbaren Säure oder Base.

9. Verbindung nach Anspruch 1, nämlich N-[2-(5-Methoxy-indol-3-yl)-ethyl]-trifluoracetamid.

10. Verbindung nach Anspruch 1, nämlich N-[2-(5-Methoxy-indol-3-yl)-ethyl]-N'-propylharnstoff.

11. Verbindung nach Anspruch 1, nämlich N-[2-(5-Methoxy-indol-3-yl)-ethyl]-N'-propylthioharnstoff.

12. Verbindung nach Anspruch 1, nämlich N-[2-(5-Methoxy-indol-3-yl)-ethyl]-cyclopropylcarboxamid.

13. Verbindung nach Anspruch 1, nämlich N-[2-(5-Methoxy-benzo[b]furan-3-yl)-ethyl]-N'-propylharnstoff.

14. Verbindung nach Anspruch 1, nämlich N-[2-(5-Methoxy-benzo[b]thiophen-3-yl)-ethyl]-N'-propylharnstoff.

15. Verbindung nach Anspruch 1, nämlich N-[2-(5-Methoxy-indol-3-yl)-ethyl]-cyclobutylcarboxamid.

16. Verbindung nach Anspruch 1, nämlich N-[2-(6-Methoxy-benzimidazol-1-yl)-ethyl]-acetamid.

17. Verbindung nach Anspruch 1, nämlich N-[2-(6-Fluor-5-methoxy-indol-3-yl)-ethyl]-cyclopropylcarboxamid.

18. Verbindung nach Anspruch 1, nämlich N-[2-(6-Fluor-5-methoxy-indol-3-yl)-ethyl]-N'-propylharnstoff.

19. Verfahren zur Herstellung der Verbindungen der Formel (I) nach Anspruch 1, **dadurch gekennzeichnet**, daß man als Ausgangsmaterial ein Amin der allgemeinen Formel (IX) einsetzt:
Ar' - CH₂CH₂ - NHR₂ (IX)
in der Ar' und R₂ die in Anspruch 1 angegebenen Bedeutungen besitzen, welches man:
- entweder mit einem Säurechlorid der Formel (X): 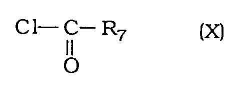 oder mit einem Säureanhydrid der Formel (XI): 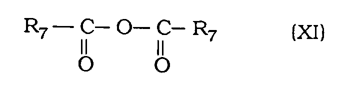 in denen R₇ die in Anspruch 1 angegebenen Bedeutungen besitzt, behandelt zur Bildung der Verbindungen der Formel (Iα): 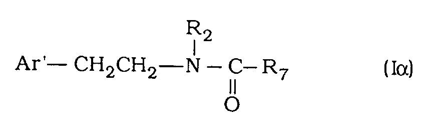 in der Ar', R₂ und R₇ die in Anspruch 1 angegebenen Bedeutungen besitzen,
- oder mit einem Isocyanat der Formel (XII):
R₈ - N = C = O (XII)
in der R₈ die in Anspruch 1 angegebenen Bedeutungen besitzt, behandelt zur Bildung der Verbindungen der Formel (Iβ): 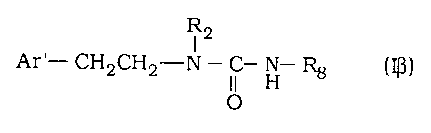 in der Ar', R₂ und R₈ die in Anspruch 1 angegebenen Bedeutungen besitzen,
- oder mit einem Isothiocyanat der Formel (XIII):
R₈ - N = C = S (XIII)
in der R₈ die in Anspruch 1 angegebenen Bedeutungen besitzt, behandelt zur Bildung der Verbindungen der Formel (Iγ): 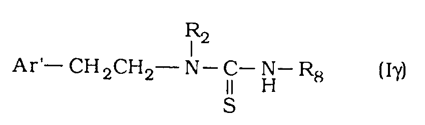 in der Ar', R₂ und R₈ die in Anspruch 1 angegebenen Bedeutungen besitzen, wobei zu verstehen ist, daß die Verbindungen der Formeln (Iα), (Iβ) und (Iγ) Gegenstand der vorliegenden Erfindung sind und die Verbindungen der Formel (I) nach Anspruch 1 bilden,
welche Verbindungen der Formel (I) nach Anspruch 1:
- mit Hilfe einer oder mehrerer Reinigungsmethoden ausgewählt aus Kristallisation, Säulenchromatographie über Siliciumdioxid, Extraktion, Filtration und Überführung über Kohlenstoff und/oder ein Harz gereinigt werden können,
- gegebenenfalls in ihre eventuellen optischen Isomeren in reiner Form oder in Form einer Mischung aufgetrennt werden können,
- und/oder mit einer pharmazeutisch annehmbaren Säure oder Base in die Salze überführt werden können.

20. Verfahren zur Herstellung der Verbindungen der Formel (Iε),einem Sonderfall der Verbindungen der Formel (I) nach Anspruch 1: 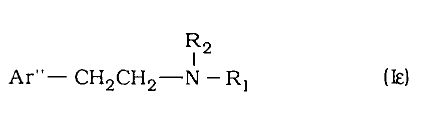 in der R₁ und R₂ die in Anspruch 1 angegebenen Bedeutungen besitzen und Ar'' eine Gruppe Ar' darstellt, wie sie in Anspruch 1 definiert ist und die durch eine Gruppe -O-R₃'' substituiert ist, worin R₃'' eine Gruppe darstellt, die ausgewählt ist aus gegebenenfalls substituierten Arylgruppen, gegebenenfalls substituierten Arylalkyl- oder Diarylalkylgruppen und Cycloalkyl- oder Cycloalkylalkylgruppen (wobei die Begriffe "Arylgruppe", Arylalkylgruppe", "Diarylalkylgruppe", "Cycloalkylgruppe", "Cycloalkylalkylgruppe" und "substituiert" den Bedeutungen entsprechen, wie sie in Anspruch 1 definiert sind),
**dadurch gekennzeichnet**, daß man
eine Verbindung der Formel (Iε'): 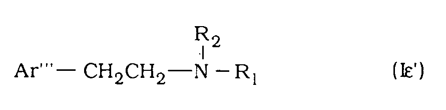 in der R₁ und R₂ die oben angegebenen Bedeutungen besitzen und Ar''' eine Gruppe Ar' darstellt, wie sie in Anspruch 1 definiert ist, die durch eine Gruppe -O-R₃''' substituiert ist, wobei R₃''' ein Wasserstoffatom bedeutet, mit einer Verbindung der Formel (XIV):
R'' - Hal (XIV)
in der Hal ein Halogenatom und R'' eine Gruppe ausgewählt aus gegebenenfalls substituierten Arylgruppen, gegebenenfalls substituierten Arylalkyl- oder Diarylalkylgruppen und Cycloalkyl- oder Cycloalkylalkylgruppen bedeuten (wobei die Begriffe "Arylgruppe", Arylalkylgruppe", "Diarylalkylgruppe", "Cycloalkylgruppe", "Cycloalkylalkylgruppe" und "substituiert" den Bedeutungen entsprechen, wie sie in Anspruch 1 definiert sind), umsetzt,
wobei die Verbindungen der Formel (Iε):
- mit Hilfe einer oder mehrerer Reinigungsmethoden ausgewählt aus Kristallisation, Säulenchromatographie über Siliciumdioxid, Extraktion, Filtration und Überführung über Kohlenstoff und/oder ein Harz gereinigt werden können,
- gegebenenfalls in ihre eventuellen optischen Isomeren in reiner Form oder in Form einer Mischung aufgetrennt werden können,
- und/oder mit einer pharmazeutisch annehmbaren Säure oder Base in die Salze überführt werden können.

21. Verfahren zur Herstellung der Verbindungen der Formel (Iφ), einem Sonderfall der Verbindungen der Formel (I) nach Anspruch 1: 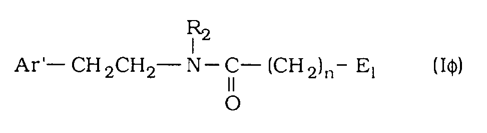 in der Ar', R₂, E₁ und n die in Anspruch 1 angegebenen Bedeutungen besitzen, **dadurch gekennzeichnet**, daß man eine Verbindung der Formel (Iφ'): 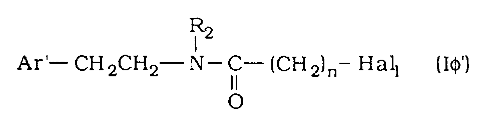 in der Ar', R₂ und n die in Anspruch 1 angegebenen Bedeutungen besitzen und Hal₁ ein Halogenatom darstellt, mit einem Morpholin oder einem Piperazin, das nichtsubstituiert oder durch eine Gruppe -(CH₂)_{n'}-E₂ substituiert ist, worin n' und E₂ die in Anspruch 1 angegebenen Bedeutungen besitzen, umsetzt,
wobei die Verbindungen der Formel (Iφ):
- mit Hilfe einer oder mehrerer Reinigungsmethoden ausgewählt aus Kristallisation, Säulenchromatographie über Siliciumdioxid, Extraktion, Filtration und Überführung über Kohlenstoff und/oder ein Harz gereinigt werden können,
- gegebenenfalls in ihre eventuellen optischen Isomeren in reiner Form oder in Form einer Mischung aufgetrennt werden können,
- und/oder mit einer pharmazeutisch annehmbaren Säure oder Base in die Salze überführt werden können.

22. Pharmazeutische Zubereitung enthaltend als Wirkstoff mindestens eine Verbindung nach einem der Ansprüche 1 bis 18 oder eines ihrer Additionssalze mit einer pharmazeutisch annehmbaren Säure oder Base allein oder in Kombination mit einem oder mehreren inerten, nichttoxischen, pharmazeutisch annehmbaren Trägermaterialien oder Bindemitteln.

23. Pharmazeutische Zubereitung nach Anspruch 22 zur Behandlung von Störungen des melatoninergischen Systems.

24. Pharmazeutische Zubereitung nach Anspruch 22 zur Behandlung von Streß, Schlafstörungen, der Angst, von jahreszeitlich bedingten Depressionen, der Schlaflosigkeit und der Müdigkeit als Folge von Zeitverschiebungen, der Schizophrenie, von Panikanfällen, der Melancholie, zur Steuerung des Appetits, der Schlaflosigkeit, von psychotischen Störungen, der Epilepsie, der Parkinsonschen Krankheit, der Altersdemenz, von Störungen, die mit dem normalen oder pathologischen Altern verbunden sind, der Migräne, von Gedächtnisverlust, der Alzheimerschen Krankheit sowie von Störungen der Gehirnzirkulation, von bestimmten Krebsen, der Psoriasis, der Akne, der Seborrhoe oder zur Inhibierung der Ovulation oder in der Veterinärmedizin bei Störungen des Fellwuchses.

## Patentansprüche (Patentansprüche für folgende(n) Vertragsstaat(en): ES, GR)

1. Verfahren zur Herstellung von Verbindungen der allgemeinen Formel (I): 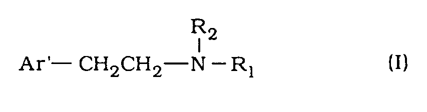 in der:
- Ar':
- einen Indol-3-yl-kern der Formel (II): 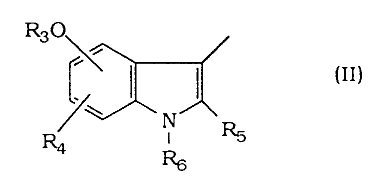
- einen Benzo[b]thiophen-3-yl-kern der Formel (III): 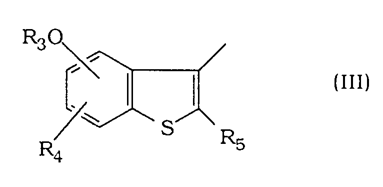
- einen Benzimidazol-1-yl-kern der Formel (IV): 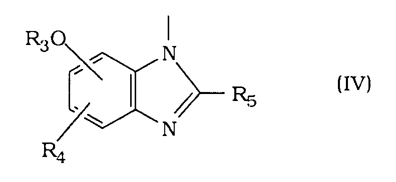
- einen Benzo[b]furan-3-yl-kern der Formel (V): 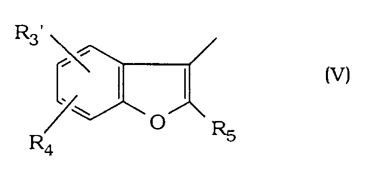
- einen 1,2-Benzisoxazol-3-yl-kern der Formel (VI): 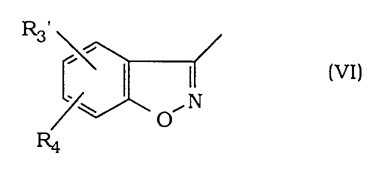
- einen 1,2-Benzisothiazol-3-yl-kern der Formel (VII): 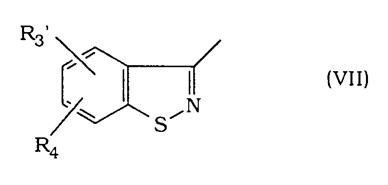 oder
- einen Indazol-3-yl-kern der Formel (VIII): 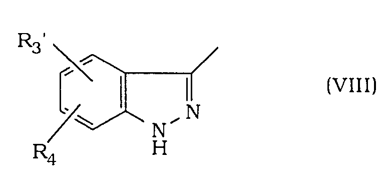
- R₁:
- eine Gruppe 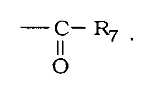 in der R₇ eine gegebenenfalls substituierte Cycloalkylgruppe, eine gegebenenfalls substituierte Cycloalkyl-(C₁-C₄)-alkylgruppe oder eine Trifluormethylgruppe darstellt,
mit der Maßgabe, daß, wenn Ar' eine Gruppe ausgewählt aus den Gruppen der Formeln (IV), (VI), (VII) und (VIII) darstellt, R₇ auch eine geradkettige oder verzweigte Alkylgruppe mit 1 bis 6 Kohlenstoffatomen, die nichtsubstituiert oder mit 1 bis 2 Halogenresten substituiert sein kann, darstellen kann,
- eine Gruppe 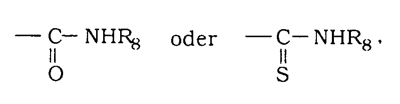 in der R₈ eine geradkettige oder verzweigte Niedrigalkylgruppe mit 1 bis 6 Kohlenstoffatomen, eine gegebenenfalls substituierte Cycloalkylgruppe, eine gegebenenfalls substituierte Cycloalkyl-(C₁-C₄)-alkylgruppe, eine gegebenenfalls substituierte Arylgruppe oder eine gegebenenfalls substituierte Arylalkylgruppe, deren Alkylkette 1 bis 3 Kohlenstoffatome aufweist, darstellt,
- eine Gruppe 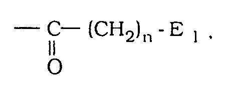 in der
n eine ganze Zahl mit einem Wert zwischen 1 und 3 und E₁ einen Rest ausgewählt aus:
- Morpholino,
- Piperazin, der nichtsubstituiert oder durch eine Gruppe -(CH₂)_{n'}-E₂ substituiert ist, worin n' eine ganze Zahl mit einem Wert von 1 bis 4 und E₂ eine Phenyl- oder Naphthylgruppe, die nichtsubstituiert oder durch einen bis drei Reste ausgewählt aus Halogenatomen, (C₁-C₄)-Alkylgruppen und (C₁-C₄)-Alkoxygruppen substituiert ist, bedeuten,
darstellen,
- R₂ ein Wasserstoffatom oder eine geradkettige oder verzweigte Niedrigalkylgruppe mit 1 bis 6 Kohlenstoffatomen,
- R₃ ein Wasserstoffatom, eine geradkettige oder verzweigte Niedrigalkylgruppe mit 1 bis 6 Kohlenstoffatomen, eine gegebenenfalls substituierte Arylgruppe, eine gegebenenfalls substituierte Arylalkyl- oder Diarylalkylgruppe, worin die Alkylkette 1 bis 3 Kohlenstoffatome aufweist, oder eine Cycloalkylgruppe oder eine Cycloalkylalkylgruppe, worin die Alkylkette 1 bis 3 Kohlenstoffatome aufweist,
- R₃' ein Wasserstoffatom oder eine Gruppe -O-R₃, worin R₃ die oben angegebenen Bedeutungen besitzt,
- R₄ ein Wasserstoffatom, ein Halogenatom, eine Hydroxylgruppe, eine geradkettige oder verzweigte Alkoxygruppe mit 1 bis 6 Kohlenstoffatomen oder eine geradkettige oder verzweigte Niedrigalkylgruppe mit 1 bis 6 Kohlenstoffatomen,
- R₅ ein Wasserstoffatom, ein Halogenatom, eine geradkettige oder verzweigte Niedrigalkylgruppe mit 1 bis 6 Kohlenstoffatomen, eine gegebenenfalls substituierte Phenylgruppe oder eine gegebenenfalls substituierte Phenylalkylgruppe, deren Alkylkette 1 bis 3 Kohlenstoffatome aufweist,
- R₆ ein Wasserstoffatom oder eine geradkettige oder verzweigte Niedrigalkylgruppe mit 1 bis 6 Kohlenstoffatomen, bedeuten,
- deren Isomere, Epimere und Diastereoisomere,
- und deren Additionssalze mit einer pharmazeutisch annehmbaren Säure oder Base,
- mit der Maßgabe, daß:
· Ar' keine 7-Methoxy-benzo[b]furan-3-yl-gruppe darstellt, wenn R₁ eine Cyclopropylcarbonylgruppe bedeutet,
· R₁ keine Trifluoracetylgruppe darstellt, wenn Ar' einen Indolrest darstellt, worin R₂ = R₃ = R₄ = R₅ = R₆ = H bedeuten,
· R₁ keine Anilinothiocarbonylgruppe darstellt, die nichtsubstituiert oder in der 4-Stellung des Phenylrests durch eine Alkoxygruppe substituiert ist, wenn Ar' einen Indol-3-yl-kern und R₃ eine Methyl- oder Benzylgruppe bedeuten,
· und R₁ keine Gruppe -CO-NH-R₈ darstellt, wenn Ar' einen Kern der Formel (II) und R₅ eine gegebenenfalls substituierte Phenylalkylgruppe, deren Alkylkette 1 bis 3 Kohlenstoffatome aufweist, bedeuten,
wobei, wenn nichts anderes angegeben ist:
- der mit den Begriffen "Arylgruppe", "Arylalkylgruppe", "Diarylalkylgruppe", "Phenylgruppe" und "Phenylalkylgruppe" verknüpfte Ausdruck "substituiert" bedeutet, daß der oder die aromatischen Kerne durch eine oder mehrere Gruppen ausgewählt aus: geradkettigen oder verzweigten Niedrigalkylgruppen mit 1 bis 6 Kohlenstoffatomen, geradkettigen oder verzweigten Niedrigalkoxygruppen mit 1 bis 6 Kohlenstoffatomen, Hydroxylgruppen, Halogenatomen, Nitrogruppen und Trifluormethylgruppen substituiert sein können,
- der mit den Bezeichnungen "Cycloalkylgruppe" und "Cycloalkyl-(C₁-C₄)-alkylgruppe" verknüpfte Ausdruck "substituiert" bedeutet, daß das cyclische System durch einen oder mehrere Reste ausgewählt aus Halogenatomen, geradkettigen oder verzweigten Niedrigalkylgruppen mit 1 bis 6 Kohlenstoffatomen und geradkettigen oder verzweigten Niedrigalkoxygruppen mit 1 bis 6 Kohlenstoffatomen substituiert sein können,
- der Begriff "Cycloalkylgruppe" für ein gesättigtes oder ungesättigtes cyclisches System mit 3 bis 8 Kohlenstoffatomen steht und
- unter einer "Arylgruppe" eine Pyridyl-, Phenyl-, Naphthyl-, Thienyl-, Furyl- oder Pyrimidylgruppe zu verstehen ist,
**dadurch gekennzeichnet**, daß man als Ausgangsmaterial ein Amin der allgemeinen Formel (IX) einsetzt:
Ar' - CH₂CH₂ - NHR₂ (IX)
in der Ar' und R₂ die bezüglich der Formel (I) angegebenen Bedeutungen besitzen, welches man:
- entweder mit einem Säurechlorid der Formel (X): 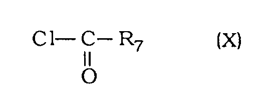 oder mit einem Säureanhydrid der Formel (XI): 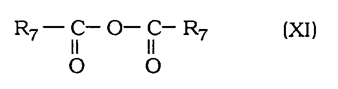 in denen R₇ die bezüglich der Formel (I) angegebenen Bedeutungen besitzt, behandelt zur Bildung der Verbindungen der Formel (Iα): 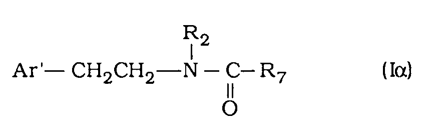 in der Ar', R₂ und R₇ die bezüglich der Formel (I) angegebenen Bedeutungen besitzen,
- oder mit einem Isocyanat der Formel (XII):
R₈ - N = C = O (XII)
in der R₈ die bezüglich der Formel (I) angegebenen Bedeutungen besitzt, behandelt zur Bildung der Verbindungen der Formel (Iβ): 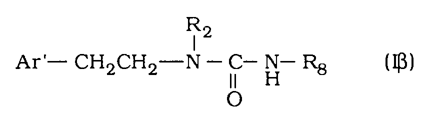 in der Ar', R₂ und R₈ die bezüglich der Formel (I) angegebenen Bedeutungen besitzen,
- oder mit einem Isothiocyanat der Formel (XIII):
R₈ - N = C = S (XIII)
in der R₈ die bezüglich der Formel (I) angegebenen Bedeutungen besitzt, behandelt zur Bildung der Verbindungen der Formel (Iγ): 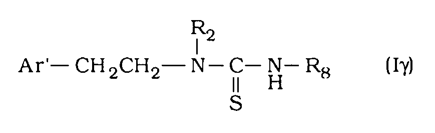 in der Ar', R₂ und R₈ die bezüglich der Formel (I) angegebenen Bedeutungen besitzen,
wobei zu verstehen ist, daß die Verbindungen der Formeln (Iα), (Iβ) und (Iγ) Gegenstand der vorliegenden Erfindung sind und die Verbindungen der Formel (I) bilden,
welche Verbindungen der Formel (I):
- mit Hilfe einer oder mehrerer Reinigungsmethoden ausgewählt aus Kristallisation, Säulenchromatographie über Siliciumdioxid, Extraktion, Filtration und Überführung über Kohlenstoff und/oder ein Harz gereinigt werden können,
- gegebenenfalls in ihre eventuellen optischen Isomeren in reiner Form oder in Form einer Mischung aufgetrennt werden können,
- und/oder mit einer pharmazeutisch annehmbaren Säure oder Base in die Salze überführt werden können.

2. Verfahren zur Herstellung der Verbindungen der Formel (Iε), einem Sonderfall der Verbindungen der Formel (I) nach Anspruch 1: 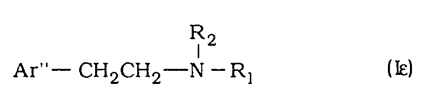 in der R₁ und R₂ die in Anspruch 1 angegebenen Bedeutungen besitzen und Ar'' eine Gruppe Ar' darstellt, wie sie in Anspruch 1 definiert ist und die durch eine Gruppe -O-R₃'' substituiert ist, worin R₃'' eine Gruppe darstellt, die ausgewählt ist aus gegebenenfalls substituierten Arylgruppen, gegebenenfalls substituierten Arylalkyl- oder Diarylalkylgruppen und Cycloalkyl- oder Cycloalkylalkylgruppen (wobei die Begriffe "Arylgruppe", Arylalkylgruppe", "Diarylalkylgruppe", "Cycloalkylgruppe", "Cycloalkylalkylgruppe" und "substituiert" den Bedeutungen entsprechen, wie sie in Anspruch 1 definiert sind),
**dadurch gekennzeichnet**, daß man
eine Verbindung der Formel (Iε'): 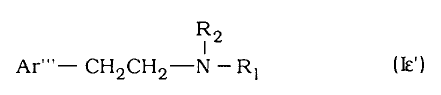 in der R₁ und R₂ die oben angegebenen Bedeutungen besitzen und Ar''' eine Gruppe Ar' darstellt, wie sie in Anspruch 1 definiert ist, die durch eine Gruppe -O-R₃''' substituiert ist, wobei R₃''' ein Wasserstoffatom bedeutet, mit einer Verbindung der Formel (XIV):
R'' - Hal (XIV)
in der Hal ein Halogenatom und R'' eine Gruppe ausgewählt aus gegebenenfalls substituierten Arylgruppen, gegebenenfalls substituierten Arylalkyl- oder Diarylalkylgruppen und Cycloalkyl- oder Cycloalkylalkylgruppen bedeuten (wobei die Begriffe "Arylgruppe", Arylalkylgruppe", "Diarylalkylgruppe", "Cycloalkylgruppe", "Cycloalkylalkylgruppe" und "substituiert" den Bedeutungen entsprechen, wie sie in Anspruch 1 definiert sind), umsetzt,
wobei die Verbindungen der Formel (Iε):
- mit Hilfe einer oder mehrerer Reinigungsmethoden ausgewählt aus Kristallisation, Säulenchromatographie über Siliciumdioxid, Extraktion, Filtration und Überführung über Kohlenstoff und/oder ein Harz gereinigt werden können,
- gegebenenfalls in ihre eventuellen optischen Isomeren in reiner Form oder in Form einer Mischung aufgetrennt werden können,
- und/oder mit einer pharmazeutisch annehmbaren Säure oder Base in die Salze überführt werden können.

3. Verfahren zur Herstellung der Verbindungen der Formel (Iφ), einem Sonderfall der Verbindungen der Formel (I) nach Anspruch 1: 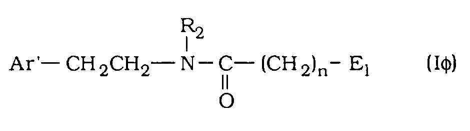 in der Ar', R₂, E₁ und n die in Anspruch 1 angegebenen Bedeutungen besitzen, **dadurch gekennzeichnet**, daß man eine Verbindung der Formel (Iφ'): 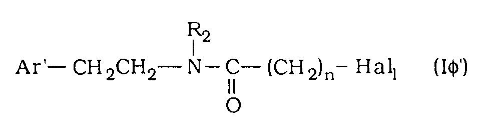 in der Ar', R₂ und n die in Anspruch 1 angegebenen Bedeutungen besitzen und Hal₁ ein Halogenatom darstellt, mit einem Morpholin oder einem Piperazin, das nichtsubstituiert oder durch eine Gruppe -(CH₂)_{n'}-E₂ substituiert ist, worin n' und E₂ die in Anspruch 1 angegebenen Bedeutungen besitzen, umsetzt,
wobei die Verbindungen der Formel (Iφ):
- mit Hilfe einer oder mehrerer Reinigungsmethoden ausgewählt aus Kristallisation, Säulenchromatographie über Siliciumdioxid, Extraktion, Filtration und Überführung über Kohlenstoff und/oder ein Harz gereinigt werden können,
- gegebenenfalls in ihre eventuellen optischen Isomeren in reiner Form oder in Form einer Mischung aufgetrennt werden können,
- und/oder mit einer pharmazeutisch annehmbaren Säure oder Base in die Salze überführt werden können.

4. Verfahren nach einem der Ansprüche 1 bis 3 zur Herstellung von Verbindungen, worin Ar' einen Indol-3-yl-kern darstellt, der Indolen der Formel entspricht: 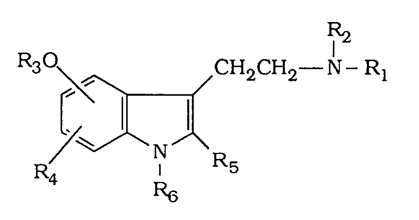 in der R₁, R₂, R₃, R₄, R₅ und R₆ die in Anspruch 1 angegebenen Bedeutungen besitzen,
und von deren Isomeren, Epimeren und Diastereoisomeren sowie von deren Additionssalzen mit einer pharmazeutisch annehmbaren Säure oder Base.

5. Verfahren nach einem der Ansprüche 1 bis 3 zur Herstellung von Verbindungen, worin Ar' einen Benzo[b]thiophen-3-yl-kern darstellt, der Benzo[b]thiophenen der Formel entspricht: 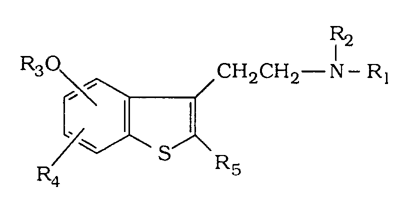 in der R₁, R₂, R₃, R₄ und R₅ die in Anspruch 1 angegebenen Bedeutungen besitzen, und von deren Isomeren, Epimeren und Diastereoisomeren sowie von deren Additionssalzen mit einer pharmazeutisch annehmbaren Säure oder Base.

6. Verfahren nach einem der Ansprüche 1 bis 3 zur Herstellung von Verbindungen, worin Ar' einen Benzo[b]furan-3-yl-kern bedeutet, der Benzo[b]furanen der Formel entspricht: 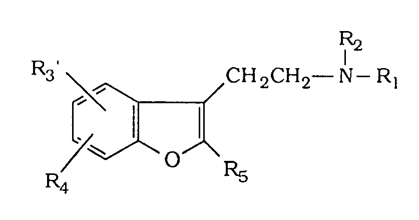 in der R₁, R₂, R₃', R₄ und R₅ die in Anspruch 1 angegebenen Bedeutungen besitzen, von deren Isomeren, Epimeren und Diastereoisomeren sowie von deren Additionssalzen mit einer pharmazeutisch annehmbaren Säure oder Base.

7. Verfahren nach einem der Ansprüche 1 bis 3 zur Herstellung von Verbindungen, worin Ar' einen Benzimidazol-1-yl-kern bedeutet, der Benzimidazolen der Formel entspricht: 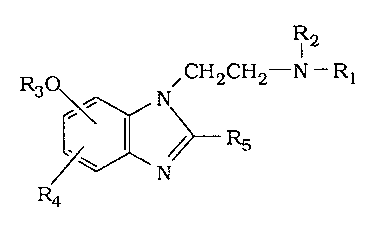 in der R₁, R₂, R₃, R₄ und R₅ die in Anspruch 1 angegebenen Bedeutungen besitzen, und von deren Isomeren, Epimeren und Diastereoisomeren sowie von deren Additionssalzen mit einer pharmazeutisch annehmbaren Säure oder Base.

8. Verfahren nach einem der Ansprüche 1 bis 3 zur Herstellung von Verbindungen, worin Ar' einen 1,2-Benzisothiazol-3-yl-kern darstellt, der 1,2-Benzisothiazolen der allgemeinen Formel entspricht: 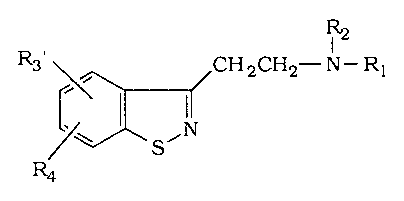 in der R₁, R₂, R₃' und R₄ die in Anspruch 1 angegebenen Bedeutungen besitzen, und von deren Isomeren, Epimeren und Diastereoisomeren sowie von deren Additionssalzen mit einer pharmazeutisch annehmbaren Säure oder Base.

9. Verfahren nach einem der Ansprüche 1 bis 3 zur Herstellung von Verbindungen, worin Ar' einen 1,2-Benzisoxazol-3-yl-kern darstellt, der Benzisoxazolen der allgemeinen Formel entspricht: 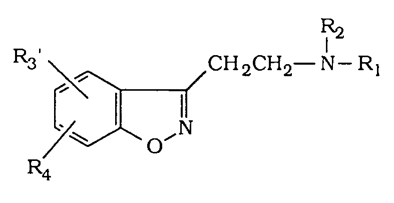 in der R₁, R₂, R₃' und R₄ die in Anspruch 1 angegebenen Bedeutungen besitzen, und von deren Isomeren, Epimeren und Diastereoisomeren sowie von deren Additionssalzen mit einer pharmazeutisch annehmbaren Säure oder Base.

10. Verfahren nach einem der Ansprüche 1 bis 3 zur Herstellung von Verbindungen, worin Ar' einen Indazol-3-yl-kern darstellt, der Indazolen der allgemeinen Formel entspricht: 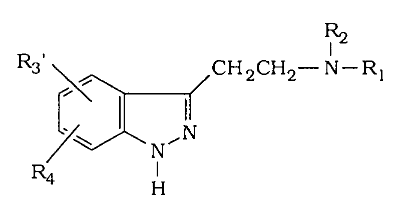 in der R₁, R₂, R₃' und R₄ die in Anspruch 1 angegebenen Bedeutungen besitzen, und von deren Isomeren, Epimeren und Diastereoisomeren sowie von deren Additionssalzen mit einer pharmazeutisch annehmbaren Säure oder Base.

11. Verfahren nach Anspruch 1 zur Herstellung von N-[2-(5-Methoxy-indol-3-yl)-ethyl]-trifluoracetamid.

12. Verfahren nach Anspruch 1 zur Herstellung von N-[2-(5-Methoxy-indol-3-yl)-ethyl]-N'-propylharnstoff.

13. Verfahren nach Anspruch 1 zur Herstellung von N-[2-(5-Methoxy-indol-3-yl)-ethyl]-N'-propylthioharnstoff.

14. Verfahren nach Anspruch 1 zur Herstellung von N-[2-(5-Methoxy-indol-3-yl)-ethyl]-cyclopropylcarboxamid.

15. Verfahren nach Anspruch 1 zur Herstellung von N-[2-(5-Methoxy-benzo[b]furan-3-yl)-ethyl]-N'-propylharnstoff.

16. Verfahren nach Anspruch 1 zur Herstellung von N-[2-(5-Methoxy-benzo[b]thiophen-3-yl)-ethyl]-N'-propylharnstoff.

17. Verfahren nach Anspruch 1 zur Herstellung von N-[2-(5-Methoxy-indol-3-yl)-ethyl]-cyclobutylcarboxamid.

18. Verfahren nach Anspruch 1 zur Herstellung von N-[2-(6-Methoxy-benzimidazol-1-yl)-ethyl]-acetamid.

19. Verfahren nach Anspruch 1 zur Herstellung von N-[2-(6-Fluor-5-methoxy-indol-3-yl)-ethyl]-cyclopropylcarboxamid.

20. Verfahren nach Anspruch 1 zur Herstellung von N-[2-(6-Fluor-5-methoxy-indol-3-yl)-ethyl]-N'-propylharnstoff.

21. Verfahren zur Herstellung einer pharmazeutischen Zubereitung, die als Wirkstoff mindestens eine Verbindung hergestellt nach einem der Ansprüche 1 bis 20 oder eines ihrer Additionssalze mit einer pharmazeutisch annehmbaren Säure oder Base allein oder in Kombination mit einem oder mehreren inerten, nichttoxischen, pharmazeutisch annehmbaren Trägermaterialien oder Bindemitteln enthält.

22. Verfahren zur Herstellung einer pharmazeutischen Zubereitung nach Anspruch 21, die zur Behandlung von Störungen des melatoninergischen Systems geeignet ist.

23. Verfahren zur Herstellung einer pharmazeutischen Zubereitung nach Anspruch 21, die zur Behandlung von Streß, Schlafstörungen, der Angst, von jahreszeitlich bedingten Depressionen, der Schlaflosigkeit und der Müdigkeit als Folge von Zeitverschiebungen, der Schizophrenie, von Panikanfällen, der Melancholie, zur Steuerung des Appetits, der Schlaflosigkeit, von psychotischen Störungen, der Epilepsie, der Parkinsonschen Krankheit, der Altersdemenz, von Störungen, die mit dem normalen oder pathologischen Altern verbunden sind, der Migräne, von Gedächtnisverlust, der Alzheimerschen Krankheit sowie von Störungen der Gehirnzirkulation, von bestimmten Krebsen, der Psoriasis, der Akne, der Seborrhoe oder zur Inhibierung der Ovulation oder in der Veterinärmedizin bei Störungen des Fellwuchses geeignet ist.
